# EUROPEAN PATENT SPECIFICATION

(11) **EP 3 003 481 B1**
(45) Date of publication and mention of the grant of the patent: **17.10.2018**
(21) Application number: 14827021.8
(22) Date of filing: 30.05.2014
(51) Int. Cl.: A61N 5/06

(54) **SELF-ADMINISTRABLE SYSTEM FOR NON-INVASIVE NEUROSTIMULATION THERAPY OF THE BRAIN**
SELBSTVERWALTENDES SYSTEM ZUR NICHTINVASIVEN NEUROSTIMULATIONSTHERAPIE DES GEHIRNS
SYSTÈME À ADMINISTRATION AUTOMATIQUE POUR UNE THÉRAPIE DE NEUROSTIMULATION NON-INVASIVE DU CERVEAU

(30) Priority: 04.06.2013 US 201313986768
(43) Date of publication of application: 13.04.2016
(73) Proprietor: Lim, Lew, Toronto, ON M1R 1W9 (CA)
(72) Inventor: Lim, Lew, Toronto, ON M1R 1W9 (CA)
(74) Representative: Bassil, Nicholas Charles
(86) International application number: PCT/IB2014/002079
(87) International publication number: WO 2015/008154

(56) References cited:
- WO-A1-2012/161394
- US-A1- 2006 287 695
- US-A1- 2007 219 600
- US-A1- 2007 219 600
- US-A1- 2008 033 512
- US-A1- 2010 324 632
- US-A1- 2012 245 659
- US-A1- 2013 035 746

## Description

### FIELD OF THE INVENTION

The invention concerns a novel method to stimulate therapeutic outcomes by irradiating the various parts brain with light - *i.e.,* neurostimulation. This is anatomically achieved through the nasal cavity (intranasal neurostimulation) by inserting a small clear plastic-encapsulated apparatus comprising a light diode that is controlled by a control assembly unit, and is powered by a replenishable dry cell battery. There is no invasion into the body tissue.

The irradiating light may be untargeted - *i.e.,* broadly directed to stimulate the general brain area; or be purposefully targeted at specific regions of the brain in order to achieve particular therapeutic outcomes. The coverage area and depth of light stimulation is influenced by selecting the appropriate wavelength of light, its direction, the effective energy output, and the coherency (as relates to a laser source). The neurostimulation apparatus and system is small and is hands-free, allowing for full mobility and portability for the user; and offers a new and easy method to stimulate the brain for therapeutic purposes. The parameters may be controlled with a small control assembly unit, or with a smart-phone having the appropriate downloaded application software.

### BACKGROUND OF THE INVENTION

### Current State Of The Art For Brain Stimulation Methods:

Brain stimulation techniques of various kinds have been part of the neuroscience world for some time, based on the fact that the neural system have always responded to these in some substantive ways. Most of these are based on electrical and magnetic impulses. The following listing constitutes the major methods currently used today to stimulate the human brain for therapeutic purposes.

### 1. Electroconvulsive Therapy (ECT)

Electroconvulsive therapy (ECT) is one of the oldest methods used to electrically induce seizure in anesthetized patients in order to treat difficult cases of severe depression, mania and catatonia [see for example, Rudorfer, MV, Henry, ME, Sackeim, HA (2003). "Electroconvulsive therapy". In Tasman A, Kay J, Lieberman JA (eds) Psychiatry, Second Edition. Chichester: John Wiley & Sons Ltd, 1865-1901].

The mechanism of action of the ECT method is still not fully understood, and there is no general consensus on the treatment protocol. Furthermore, the ECT method carries the risk of damaging the brain, such injury being represented by cognitive deficits [see for example, Breggin P (2007). "ECT Damages the Brain: Disturbing News for Patients and Shock Doctors Alike". Ethical Human Psychology and Psychiatry. 9(2): 83-86]. In addition, the consequent loss in IQ and memory from the therapy is also significant [see Andre L (2009). "Doctorsof Deception: What They Don't Want You to Know About Shock Treatment". Rutgers University Press].

### 2. Cranial Electrotherapy Stimulation (CES)

Another electrical brain stimulation technique involves cranial electrotherapy stimulation ("CES"). This CES method applies a small pulsed electric current across the patient's head. Some medical practitioners claim that CES helps with conditions such as stress, anxiety, depression and insomnia - but it is still an experimental technique [see for example, Klawansky S (1995). "Meta-Analysis of Randomized Controlled Trials of Cranial Electrostimulation: Efficacy in Treating Selected Psychological and Physiological Constitutions". Journal of Nervous & Mental Disease 183(7):478-484].

The proposed mechanism of action for CES is that the pulses of electric current increase the ability of the neural cells to produce serotonin, dopamine, DHEA, endorphins and other neurotransmitters that stabilize the neurohormonal systems [see Gilula MF, Kirsch DL (2005). "Cranial Electrotherapy Stimulation Review: A Safer Alternative to Psychopharmaceuticals in the Treatment of Depression". Journal of Neurotherapy. 9(2)]. Skeptics believe that CES may help relieve certain stress-related symptoms but has not been studied sufficiently to determine
whether its use is practical and cost-effective [see Barrett S (2008). "Dubious Claims Made for NutriPax and Cranial Electrotherapy Stimulation". QuackWatch online, extracted on May 2012]*.*

### 3. Deep Brain Stimulation (DBS)

Deep brain stimulation (DBS) utilizes implants which function by delivering measured doses of electrical stimulation via a thin electrode surgically inserted through a small hole in a patient's skull, with its tip implanted in a targeted brain area. The U.S. Food and Drug Administration (FDA) has approved these devices and procedures for treatment of a disorder called "essential tremor" in 1997; for treatment of Parkinson's disease in 2002; and for treatment of dystonia in 2003 [see Kringelbach ML, Jenkinson N, Owen SLF, Aziz TZ (2007). "Translational principles of deep brain stimulation". Nature Reviews Neuroscience. 8:623-635]. Lately, Alzheimer's Disease is reported to also respond to DBS [see Wood J (2012). "Brain Pacemaker Shows Promise in Fighting Alzheimer's Disease". PSychCentral.com online (May 12, 2012]*.*

Despite their success, DBS treatments can be overactive in its effects - an outcome which can trigger dizziness, tingling, and other undesirable side effects. Researchers also still do not understand how DBS treatment actually works in-vivo.

### 4. Transcranial Light Therapy (TLT)

Transcranial light therapy ("TLT") is enjoying more attention in recent years due to sound scientific principles, successful outcomes, lack of side-effects and being non-invasive. This method involves directing light to the brain through outside of the skull. The source of light can be light emitting diodes (LED) or a low level laser source, usually in the red or near infrared-red (NIR) part of the spectrum. The NIR band would be the preferred choice in order to provide deeper penetration through the meninges, cranial material and then through the brain matter, in order to reach the deeper parts of the brain.

Recent research supports transcranial light therapy's potential for treating stroke, traumatic brain injury, Parkinson's disease, mild cognitive impairment, Alzheimer's disease, depression, and some other cognitive issues [see for example, Rojas JC, Gonzalez-Lima F (2011). "Low-level light therapy of the eye and brain". Eye and Brain. 3:49-67]. More recently, it has also been found that this modality can also enhance cortical metabolic capacity and retention of extinction memories, reduce fear renewal, and implicate low level light therapy as a novel intervention to improve memory [Rojas JC et al (2012). "Low-level light therapy improves cortical metabolic capacity and memory retention". J Alzheimers Dis. 2012;32(3):741-52].

TLT devices are available commercially. The most commonly available design comprises a hand-held piece about the size of a hair dryer that contains the lens, and is connected by optic fiber to a control unit about the size of a small printer. The hand-held piece can also be in the form of a "cluster head" containing an array of LED diodes.

### 5. Ear Canal Transcranial Light Therapy

Ear canal transcranial light therapy was developed following a recent study in Finland that demonstrated that when bright light is directed into the ears, it helps to treat seasonal affective disorder (SAD) or winter depression [Timonen M et al (2012). "Can transcranial brain-targeted bright light treatment via ear canals be effective in relieving symptoms in seasonal affective disorder?". Medical Hypothesis. 78(4):511-515].

The commercially sold device has diodes in the form of ear buds with very bright white LED attached by cables to a controller unit. It is consumer-friendly and appears effective for SAD.

### 6. Optogenetic Neurostimulation (Optogenetics)

In the optogenetic neurostimulation (optogenetics) process, researchers first introduce a gene for a light-sensitive molecule, called channel rhodopsin 2 (ChR2), into a specific subset of neurons. Shining blue light on these neurons then causes them to fire. One advantage of this approach is its specificity - *i.e.,* only the neurons with the gene are activated.

This process also provides a way to shut neurons off - by introducing a different molecule, halorhodopsin ("NpHR") which uses the energy of yellow light to silence the cells. The combination of these elements makes the technique very exact in achieving specific neuro-outcomes.

Research with optogenetics can draw important understanding in relating anatomical locations of the brain with predictable behavioral outcomes. The exactness of how behaviour can be manipulated has great appeal in advancing neuroscience. However, at this time, the challenge is to translate animal experiments to human applications.

Thus, the following applies: The technique is still very much in the laboratory domain, involving small animals (mainly rats and mice). It is an invasive method involving implanting a light probe inserted into the brain, connecting from the targeted brain area to a controller unit via a catheter holding an optic fiber. To achieve precise stimulatory outcomes, it also requires the introduction of ChR2 into the specific areas of the brain to have the desired neurons fire.

In 2010, optogenetics was chosen as the 'Method of the Year' across all fields of science and engineering by the interdisciplinary research journal *Nature.* At the same time, optogenetics was highlighted in the article on "Breakthroughs of the Decade" in the scientific research journal *Science.* The precision of the optogenetics method is highly appealing to scientists, but it is expected to stay in the research laboratory domain for the foreseeable future. Today, over 500 laboratories are applying optogenetic tools to animal models of Parkinson's, blindness, spinal injury, depression, narcolepsy, addiction, and memory [see Williams M (2010). "A brain implant that uses light". Technology Review online article published February 24, 2010*].*

### Summary Of The Current State Of The Art:

There are good data supporting the efficacy of all these conventionally known methods, thereby confirming that the brain responds to light, and brings about therapeutic outcomes in various forms. However, they are all very different ways of stimulating the brain for therapeutic purposes.

Equally important, most treatment methods are either deployed in laboratory conditions on animals; or if deployed on human beings, largely have to be administered under clinical supervision. Also, the methodology that has the potential to treat a wide range of medical conditions, the transcranial method, has yet to reach a state where it can be portable and mass produced at a low cost; and thus offer the convenience of a consumer-friendly product. Thus, it suffers some disadvantages by way of mass appeal.

The optogenetics method understandably has attracted a great amount of attention in the neuoscience circle because of the exactness in which it can extract neural outcomes through precise anatomical manipulation of the brain. However, the invasiveness and set-up required keeps it in the laboratory domain. The conventional method to date that has the potential to become a consumer-friendly product, the ear canal transcranial method, is employed solely and specifically for treating seasonal affective disorder.

### The Intranasal Pathway As A Treatment Solution:

The intranasal method directing light to the brain through the nasal cavity for healing or "photobiomodulation" has the potential to overcome the disadvantages of the present brain stimulation methods. Conceptually, it would not previously have been seen feasible to pack the features necessary to achieve effective brain stimulation through the nasal cavity: The apparent limiting factors being the limited size of the human nostrils, the cosmetically unappealing thought of an intrusion into the nostrils, and the unexplored use of the nasal pathway for this purpose.

However, as a medical intervention and mode of therapeutic treatment, it should enjoy all the credibility of the transcranial method because the underlying principles are much the same; and the major differences lies in the chosen delivery pathway. Furthermore, if the light-emitting apparatus is designed properly and is small enough in its dimensions, the intranasal irradiation technique takes full advantage of the relative lack of tissue barriers between the light-emitting source and the anatomical human brain as a whole. This results in a new and unexpected treatment solution that offers safety, efficacy, low-power and portability. In addition, given an appropriate design and proper engineering of this intranasal light-emitting source, the versatility of this treatment method can offer a wide range of variability in brain stimulation effects which were neither possible nor expected previously in this technical field.

In summary, based on the current state of the art, an intranasal light irradiation method is a novel and unforeseen approach to achieve brain stimulation. This method of therapeutic treatment offers the fullest range of beneficial outcomes and yet provides the full convenience of a portable
consumer-friendly product. In this context, reference is made to document US2010/0324632 A1 relating to intranasal light therapy.

### SUMMARY OF THE INVENTION

The invention is defined in the appended set of claims. Methods mentioned hereinafter do not form part of the invention.

The present invention relates to several aspects:
A first aspect provides a self-administrable on-demand method for performing non-invasive neurostimulation therapy of the brain via a nasal cavity of a living mammalian, said self-administrable non-invasive neurostimulation method comprising the steps of obtaining a light energy-emitting apparatus comprised of
a configured irradiation lens including
   a portable hollow casing having fixed dimensions, a sized internal spatial volume, and an external surface configuration suitable for in-vivo insertion into the nasal cavity space of a nostril without causing substantial impairment to the subject's ability to breathe and without invading the nasal tissues of the living subject, said portable casing being comprised of
      (i) a light energy transmitting material which forms at least a portion of the configured external surface for said hollow casing,
      (ii) at least one light generating unit entirely housed and contained within said internal spatial volume of said hollow casing and which is capable of generating light energy of at least one preselected wavelength selected from the group consisting of near infrared red light wavelengths and visible red light wavelengths, at a predetermined energy intensity and for a preset time duration on-demand sufficient to penetrate through the nasal tissues and to pass into the brain,
   whereby said configured irradiation lens can emit light energy in any desired direction within the nasal cavity after in-vivo insertion and achieve passage of said emitted light energy from the nasal cavity into at least one portion of the brain in-vivo;
self-administrable applicator means adapted for support of said configured irradiation lens and for at will placement of said light transmitting external surface of said configured irradiation lens at a fixed position and desired irradiation direction within a nostril adjacent to the internal lining of a subject's nasal cavity;
a portable controller assembly able to control on-demand delivery of light energy from said configured irradiation lens through the subject's nasal tissues into at least one portion of the brain in-vivo, said controller assembly including
   (α) a portable and replenishable power source of on-demand direct electrical current,
   (β) a central processing unit for controlling and directing the flow of such direct electrical current, and
   (γ) at least one connector in electrical communication with said configured irradiation lens for on-demand conveyance of direct electrical current from said controller assembly to said light generating unit;
placing a transparent external surface of said configured irradiation lens within a nostril at a desired fixed position adjacent to the internal lining of a subject's nasal cavity such that light energy emitted by said configured irradiation lens will penetrate through the subject's nasal tissues and pass into at least one portion of the brain in-vivo; and
causing said light generating unit of said positioned configured irradiation lens to generate light energy of at least one preselected wavelength selected from the group consisting of near infra red light wavelengths and red light wavelengths, at a predetermined energy intensity and for a preset time duration on-demand sufficient to penetrate through the subject's nasal tissues and to pass into the brain such that neurostimulation of at least one portion of the brain is achieved.

A second aspect of the invention is a self-administrable on-demand system for performing non-invasive neurostimulation therapy of the brain via a nasal cavity of a living mammalian, said self-administrable non-invasive neurostimulation system comprising:
a configured irradiation lens including
   a portable hollow casing having fixed dimensions, a sized internal spatial volume, and an external surface configuration suitable for in-vivo insertion into the nasal cavity space of a nostril without causing substantial impairment to the subject's ability to breathe and without invading the nasal tissues of the living subject, said portable casing being comprised of
   (i) a light energy transmitting material which forms at least a portion of the configured external surface for said hollow casing,
   (ii) at least one light generating unit entirely housed and contained within said internal spatial volume of said hollow casing and which is capable of generating light energy of at least one preselected wavelength selected from the group consisting of near infrared red light wavelengths and visible red light wavelengths, at a predetermined energy intensity and for a preset time duration on-demand sufficient to penetrate through the nasal tissues and to pass into the brain,
   whereby said configured irradiation lens can emit light energy in any desired direction within the nasal cavity after in-vivo insertion and achieve passage of said emitted light energy from the nasal cavity into at least one portion of the brain in-vivo;
self-administrable applicator means adapted for support of said configured irradiation lens and for at will placement of said light transmitting external surface of said configured irradiation lens at a fixed position and desired irradiation direction within a nostril adjacent to the internal lining of a subject's nasal cavity;
a portable controller assembly able to control on-demand delivery of light energy from said configured irradiation lens through the subject's nasal tissues into at least one portion of the brain in-vivo, said controller assembly including
   (α) a portable and replenishable power source of on-demand direct electrical current,
   (β) a central processing unit for controlling and directing the flow of such direct electrical current, and
   (γ) at least one connector in electrical communication with said configured irradiation lens for on-demand conveyance of direct electrical current from said controller assembly to said light generating unit.

A third aspect of the invention provides a self-administrable on-demand dedicated apparatus for performing non-invasive neurostimulation therapy of the brain via a nasal cavity of a living mammalian, said self-administrable dedicated apparatus comprising:
a portable hollow casing having fixed dimensions, a sized internal spatial volume, and an external surface configuration suitable for in-vivo insertion into the nasal cavity space of a nostril without causing substantial impairment to the subject's ability to breathe and without invading the nasal tissues of the living subject, said portable casing being comprised of
   (i) a light energy transmitting material which forms at least a portion of the configured external surface for said hollow casing,
   (ii) at least one light generating unit entirely housed and contained within said internal spatial volume of said hollow casing and which is capable of generating light energy of at least one preselected wavelength selected from the group consisting of near infrared red light wavelengths and visible red light wavelengths, at a predetermined energy intensity and for a preset time duration on-demand sufficient to penetrate through the nasal tissues and to pass into the brain,
whereby said configured irradiation lens can emit light energy in any desired direction within the nasal cavity after in-vivo insertion and achieve passage of said emitted light energy from the nasal cavity into at least one portion of the brain in-vivo;
self-administrable applicator means adapted for support of said configured irradiation lens and for at will placement of said light transmitting external surface of said configured irradiation lens at a fixed position and desired irradiation direction within a nostril adjacent to the internal lining of a subject's nasal cavity; and
a portable controller assembly able to control on-demand delivery of light energy from said configured irradiation lens through the subject's nasal tissues into at least one portion of the brain in-vivo, said controller assembly including
   (α) a portable and replenishable power source of on-demand direct electrical current,
   (β) a central processing unit for controlling and directing the flow of such direct electrical current,
   (γ) at least one connector in electrical communication with said configured irradiation lens for on-demand conveyance of direct electrical current from said controller assembly to said light generating unit;
at least one connector in electrical communication with said source of electrical current for on-demand transfer of direct electrical current to said controller assembly; and
at least one connector in electrical communication with said controller assembly and said light generating unit housed within said hollow casing for on-demand conveyance of direct electrical current from said controller assembly.

### BRIEF DESCRIPTION OF THE FIGURES

The present invention may be easily understood and more readily appreciated when taken in conjunction with the accompanying Drawing, in which:
Fig. 1 illustrates the concept on how light from the system irradiates the brain in general, with little tissue barrier in between;
Fig. 2 illustrates the concept of how visible red light from a light emitting diode (LED) would penetrate the brain, notably with a large footprint;
Fig. 3 illustrates the concept of the relative depth of penetration by visible red light from a low level laser diode penetrating the brain, but with a smaller footprint, when compared with the LED as illustrated in Fig. 2;
Fig. 4 illustrates the concept of the relative depth and spread of penetration by near infrared red (NIR) light from a LED diode penetrating the brain when compared with the visible red LED as illustrated in Fig. 2 and visible red low level laser as illustrated in Fig. 3;
Fig. 5 is a photograph showing a preferred embodiment of the apparatus comprising the present invention;
Fig. 6 shows a preferred embodiment of the apparatus comprising the present invention;
Figs. 7a and 7b illustrate the set form version of the applicator and the cantilever based version of the applicator respectively;
Figs. 8a and 8b are engineering drawings of the side and top views respectively of the applicator assembly;
Figs. 9a and 9b are engineering drawings of the side and top views respectively of the L-shaped transparent lens unit of the applicator;
Fig 10 illustrates the positioning of the applicator of the apparatus, secured by the nose clip and shows the direction of the emitted light rays;
Fig. 11 illustrates how the nose clip of the apparatus minimizes leakage of light and redirects them back into the nasal cavity and towards the brain- as well as how the cantilever is manipulated to enable the irradiation lens is slipped into the nostril;
Fig. 12 further illustrates the role of the positioning of the diode, the micro-lens, the irradiation lens and the nose clip of the apparatus in influencing the direction of the light;
Figs. 13a and 13b illustrate alternate views of the light emitting diode embodiment of the applicator;
Figs. 14a-14d illustrate alternative views of the external form factor for the controller assembly;
Figs. 15a-15f illustrate alternative sectional views of the key internal components of the controller assembly; and
Fig. 16 illustrates the concept of the smart phone as the alternative to the controller assembly; and
Fig. 17 presents the flow chart of Table 2 and shows the intracellular mechanism of low level light therapy.

### DETAILED DESCRIPTION OF THE INVENTION

The present invention relates to a portable non-invasive apparatus, system, and method for performing irradiation light therapy upon the brain tissues through the nostrils of a living mammalian subject for the medical purpose of stimulating the brain in-vivo.

Conventionally today, there exist several known ways of stimulating the brain, but these techniques largely involve research experiments on laboratory animals or are procedures which need to be clinically/medically supervised. More recent studies on therapeutic stimulation of the brain of human beings have centered upon the transcranial light therapy methods, a technique which involves irradiating the brain from the outside of the skull.

In marked difference to these conventionally known therapeutic procedures, the present invention utilizes the intranasal pathway as the point of anatomic access; and follows established principles for the conceptual approach that irradiation of the brain tissue with light energy of certain fixed parameters would achieve therapeutic effects in-vivo. In this manner, the present invention utilizes light energy of specified intensity, wavelengths, coherency, duration and pulsed mode to achieve therapeutic outcomes.

The method, system and apparatus provided herein deliver the therapeutic light irradiation to the brain through the tissues lying adjacent to the nasal cavity. For this purpose, the light energy-emitting apparatus comprises a configured irradiation lens including a portable hollow casing having fixed dimensions, a sized internal spatial volume, and an external surface configuration suitable for in-vivo insertion into the nasal cavity space of a nostril. The nasal insertion apparatus components are designed to be small and comfortable; and to avoid causing significant or meaningful impairment in the user's ability to breathe. After insertion, the apparatus may be adjusted or preset to: direct the irradiating angle of the release of the light emitted from the apparatus, set the desired power levels, generate a pulsed frequency for the emitted light, and choose the time duration for the treatment session in order to achieve the intended therapeutic effects.

Accordingly, the treatment system of the invention takes into account that the different parameters have different therapeutic effects. As a start, it will be appreciated that the invention offers three different alternative modes produce different sets of medical outcomes.

### Major Advantages And Marked Benefits:

The instant invention provides a number of outcomes. These include the following:
- The treatment method and system utilize principles and apply operational parameters that are factually supported and evidenced by published scientific research.
- There are no known major side effects or complications associated with this treatment method and system.
- The apparatus is medically non-invasive and self-administrable in each and every embodiment and instance of use.
- The apparatus is dedicated to and designed for at will self-attachment to and self-detachment from the subject's nose.
- The apparatus is compact and collectively is only slightly larger than a matchbox.
- The apparatus is extremely light in weight, portable, and is easily transported by hand over any distance.
- The apparatus is comfortable and easy to use.
- The apparatus is to be self-administered on-demand routinely and repetitiously by the patient himself for therapeutic treatment, and does not require any assistance by a medical technician or physician.
- The apparatus can alternatively employ either a laser or a light emitting diode as a light generating unit.
- The apparatus can generate light energy waves and particles at any desired medically effective wavelength(s) chosen from the near-infrared and red light ranges.
- The apparatus causes no significant electromagnetic or other interference with other medical devices, and thus is suitable for use by persons having an implanted pacemaker or defibrillator.
- Preferred embodiments of the apparatus deploy a low level laser diode with 655 nm, or light emitting diode ("LED") diodes with 633 nm or 810 nm generated light.
- The apparatus is regulated in power to provide only about 1-20 mW; and preferably uses a 1.5 volt battery as a source of direct electric current. The power and battery requirements are specific to the light source which may be a laser or non-laser light emitting diode.
- Preferred embodiments may deliver pulsed light where this has been tested to support more therapeutic benefits for certain medical conditions.
- Preferred embodiments of the apparatus are simple to use by any person in that one merely uses his fingers to clip the applicator onto a nostril and presses the "power on" button.
- Preferred embodiments of the apparatus include both a timer and an automatic shut-off switch which self-engages after 25 minutes or other lengths of time.
- The preferred treatment duration is medically relevant; and the actual treatment time may vary depending on the choice of light source and targeted fluent (or dosage).
- Preferred embodiments of the apparatus are highly resistant to accidental injury, and are able to withstand a drop of 5 feet without incurring any damage.
- Preferred embodiments of the apparatus employ a process controller assembly which ensures that the light energy delivered to the nasal cavity is consistent. If the battery is unable to sustain a consistent power to drive the circuit to power the light source, the process controller assembly will give a warning and will switch off the device.
- Alternative embodiments include the use of "smart phones" with downloaded software applications, that perform in lieu of the process control assembly.

### I. The Underlying Premises Of The Invention

The underlying medical premises and clinical support for the principles of the present invention are demonstrated and evidenced in published neuroscientific research studies. The selected factual summaries given below provide an accurate context and proper perspectives for understanding and appreciating the true merits of the instant invention as regards neurostimulation of the living brain.

### Potential Impact And Value Of Intranasal Irradiation:

### 1. Specifically Targeted Areas Of The Brain

Specific anatomical parts of the brain govern specific functions of the mind and body. For example, the diencephalon (roughly around the mid-brain) region is the seat of some of the most essential survival functions, and holds some keys to the physical well-being of the person. This is a hard-to-reach region for access from the outside the skull but is more easily reached by a light source in the nasal cavity.

Among the anatomical brain components here, the hypothalamus is the control center for many autonomic functions. It is connected with the structures of the endocrine and parasympathetic nervous systems to support its vital role in maintaining homeostasis throughout the body. It is part of the limbic system that influences various emotional and pleasure responses, storing memories, regulating hormones, sensory perception, motor function, and olfaction. The other components of the limbic system are the amygdala, cingulated gyrus, hippocampus, olfactory cortex and the thalamus.

Whilst the mid-brain area could be a primary target, the divergent light rays will also illuminate some of the other parts of the brain to achieve a wider-spread benefit. The hypothesis of how the therapy is distributed, throughout the brain as the next stage into the secondary areas, is also based on the ability of the neural system to carry signals rapidly in its network.

The light energy may be manipulated to point towards targeted parts of the brain for more potency in specific primary areas. For example the substantia nigra (its dysfunction is attributable to Parkinson's disease) located at the bottom of the mid-brain area; or in another case, the prefrontal cortex in a separate location could be targeted to improve higher order cognitive functions and balance out primal emotions.

### 2. The Anatomical Advantages Of Intranasal Light Energy Irradiation

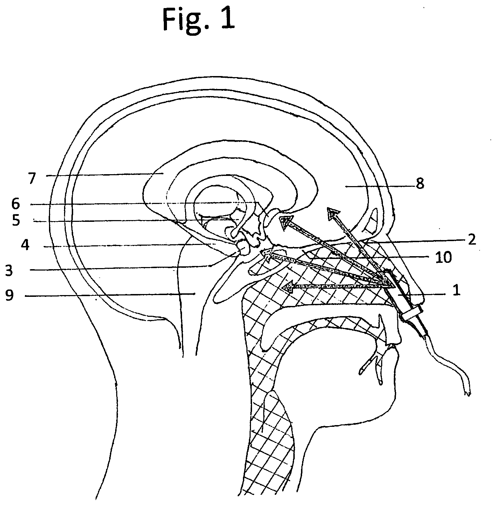

When compared to conventionally known treatment methods that endeavor to irradiate the brain in-vivo, a light source that is inserted into the nasal cavity will anatomically lie in close proximity (about 3 inches of mainly air cavity and soft tissue) to the mid-brain area. See Fig. 1.

As shown in Fig. 1, when the light source 1 in this intranasal position is pointed towards the mid-brain area, little energy is required for effective light irradiation because much of the physical pathway distance to the brain tissue is the air cavity 2 of the nostril. For purposes of illustration, the mid-brain areas highlighted anatomically are: the amygdala 3, the hippocampus 4, the hypothalamus 5, the septal area 6, and the cingulated cortex 7. The portion of the neo-cortex region that is easily illuminated by the light source is the prefrontal cortex 8.

Unquestionably, the brain itself is encased inside a bony skull. Other than the area of the brain stem 9 which connects the spinal cord to the brain, the thinnest part of the protective skull is the thin perpendicular plate of the ethmoid bone 10. As historical testament to its low barrier resistance to the brain, the ethmoid bone 10 is also that part of the skull which is typically broken during the ancient Egyptian mummification process to drain out the brain materials. Granted, there is some tissue material present as part of the nasal septum wall in the pathway leading to the mid-brain region, but such tissue material is of low density.

Having little tissue material existing between the intranasally positioned light source and the targeted brain areas is notable because red light waves and infrared red light waves penetration (as defined by the Beer-Lambert law) can suffer optical power decay of up to 80% at 1 mm distances from the surface [see for example, Abdo A, Sahin M (2007). "NIR light penetration depth in the rat peripheral nerve and brain cortex". Conf Proc IEEE Eng Med Biol Soc 2007:1723-1725]*.* Also, based on 810 nm low level laser irradiation done transcranially on mice, the average penetrative power was empirically measured; and the resulting data showed that only 15% of the of the laser power was transmitted through the skin, and only 6% of that reduced quantity penetrated through a combination of skin and skull - despite the relatively better penetrative quality of the 810 nm wavelength [see Ando T et al (2011). "Comparison of Therapeutic Effects between Pulsed and Continuous Wave 810 nm Wavelength Laser Irradiation for Traumatic Brain Injury in Mice". Plos One. 6(10)].

Anatomically, the intranasal pathway of light irradiation mainly has the much thinner perpendicular plate of the ethmoid bone existing between the brain and the light source; and there is little else of tissue consequence intervening over the distance - hence allowing more light energy penetration into the brain, given that all other operational parameters remain the same.

### 3. The Engineering Advantages Of Intranasal Light Irradiation

The anatomical advantages described above therefore allow for a therapeutic treatment system that employs a low energy, single diode with the appropriate light wavelength source pointing in the proper anatomic direction, as illustrated by Fig. 1. Thus, after this apparatus is constructed, it becomes the basis for a system and method of therapeutic treatment which is self-administrable, non-invasive, and small and convenient to use.

The control unit for managing the light generating source and light energy emissions can be miniaturized to allow for a portable and personal use system. This methodology and system has many distinct advantages over alternative modes of treatment currently available; it provides therapeutic effectiveness, a low energy demand, personal convenience, a self-administration capability, a very modest cost, and an exceptionally easy mode of use.

### II. Scientific Bases and Evidence for Brain Irradiation Therapy: Recent Relevant Research Evidence:

Because of the ineffectiveness of drugs in addressing many neurological disorders, increasing attention is being directed to alternative treatments, such as light therapy.

A summary of recent research studies (using the conventionally known transcrancial light therapy technique) which clearly shows and factually evidences the variety of beneficial in-vivo effects of low-level light therapy on the brain is provided by Table 1 below. The wide range and variety of beneficial effects is particularly noteworthy.

Furthermore, in animal research studies, low-level light therapy has been found to be promising for treating anoxic brain injury, atherothrombotic stroke, embolic stroke, Parkinson's Disease, mild cognitive impairment and Alzheimer's Disease. Similarly, in human studies, low-level light therapy has been found to be promising for improving on the effects of ischemic stroke, traumatic brain injury, depression and functions of the prefrontal cortex.

**Table 1**

| Beneficial in vivo transcranial effects of low-level light therapy on the brain | | | | | |
|---|---|---|---|---|---|
| Source | Wave-length | Dose | Effect | Relevance | References |
| Laser | 808 nm | 1.6 w/cm2, 4320J/cm2 | Increased cerebral blood flow and decreased hippocampal and cortical neuronal death after unilateral BCCAO (mouse) | Anoxic brain injury | Uozumi et al¹ |
| Laser | 808 nm | 7.5 mw/cm2, 0.9 J/cm2, 2 minutes per point | improved neurological recovery, increased subventricular neural proliferation after MCAO (rat) | Atherothromb otic stroke | DeTaboada et al,² Oron et al³ |
| Laser | 803 nm | 25 mw/cm2, 15,000 J/cm2, continuous | Improved motor function and reduction in effective dot dose for stroke 3 hours after clot injection (rabbit) | Embolic stroke | Lapchak et al⁴ |
| Laser | 808 nm | 25 mw/cm2, 15,000 J/cm2, pulsed at 1 KHz | Increased cortical ATP, decreased effective clot dose for stroke 6 hours after clot injection (rabbit) | Embolic stroke | Lapchak et al^{5 6 7} |
| Laser | 808 nm | 1J/cm2 per point | Improved clinical outcome at 90 days after ischemic stroke (human) | Ischemic stroke | Lampl et al⁸ |
| laser | 808 nm | 10 or 20 mw/cm2, 1-2-2.4 J/cm2, single point for 2 minutes | Improved motor behavior 5 days after closed-head injury, and decreased brain lesion size from 12.1% to 1.4% at 28 days after injury (mouse) | Traumatic brain injury (acute) | Oron et al⁹ |
| LED | 633 and 870 nm LED cluster | 22.2 mw/cm2, 13.3 J/cm2, 10 minutes per placement | Improved cognition of two patients with chronic mild traumatic brain injury after 2-4 months of treatment (human) | Traumatic brain injury (chronic) | Naeser et al¹⁰ |
| Laser | 670 nm | 40 mw/cm2, 2 J/cm2 in four fractions | Reduction in *substantia nigra* dopaminergic cell loss after MPTP toxicity (mouse) | Parkinson's disease | Shaw et al¹¹ |
| Laser | 1072 nm | 6 minutes × 10 days | Improved acquisition of working memory for spatial navigation in middle-aged mice (mouse) | Mild cognitive impairment, Alzheimer's disease | Michalikova et al¹² |
| LED | 810 nm | 250 mw/cm2. 60 J/cm | Decreased depression scores, increased prefrontal blood flow (human) | Depression, prefrontal functions | Schiffer et al¹³ |
| LED | 660 nm | Various at low dose, 5-11 J/cm2 | Enhanced extinction memory, reduced fear memory, enhanced cortical performance and memory (rats) | PTSD, cortical performance, memory | Rojas et al¹⁴ |

| | | | | | |
|---|---|---|---|---|---|
| Abbreviations: ATP, adenosine triphosphate; BCCAO, bilateral common carotid artery occlusion; LED, light-emitting device; MCAO, middle cerebral artery occlusion; MPTP, 1-methyl-4-phenyl-1,2,3,6-tetrahydropyridine. | | | | | |

### References

¹Uozumi Y, Nawashiro H, Sato S, Kawauchi S, Shima K, Kikuchi M (2010). "Targeted increase in cerebral blood flow by transcranial near-infrared laser irradiation". Lasers Surg Med. 42(6):566-576.
²Detaboada L, IlicS, Leichliter-Martha S, Oron U, Oron A, Streeter J (2006). "Transcranial application of low-energy laser irradiation improves neurological deficits in rats following acute stroke". Lasers Surg Med. 38(1):70-73.
³Oron A, Oron U, Chen J, et al (2006). "low-level laser therapy applied transcranially to rats after induction of stroke significantly reduces long-term neurological deficits". Stroke. 37(10):2620-2624.
⁴Lapchak PA, Wei J, Zivin JA (2004). "Transcranial infrared laser therapy improves clinical rating scores after embolic strokes in rabbits". Stroke. 35(8):1985-1988.
⁵Lapchak PA, Salgado KF, Chao CH, Zivin JÁ (2007). "Transcranial near-infrared light therapy improves motor function following embolic strokes in rabbits: an extended therapeutic window study using continuous and pulse frequency delivery modes". Neuroscience. 148(4):907-914.
⁶Lapchak PA, Han MK, Salgado KF, Streeter J, Zivin JA (2008). "Safety profile of transcranial near-infrared laser therapy administered in combination with thrombolytic therapy to embolized rabbits". Stroke. 39(11):3073-3078.
⁷Lapchak PA, De Taboada L (2010). "Transcranial near infrared laser treatment (NILT) increases cortical adenosine-5'-triphosphate (ATP) content following embolic strokes in rabbits". Brain Res. 1306:100-105.
⁸Lampl Y, Zivin JA, Fisher M, et al (2007). « Infrared laser therapy for ischemic stroke: a new treatment strategy: results of the NeuroThera Effectiveness and Safety Trial-1 (NEST-1). Stroke.38(6):1843-1849.
⁹Oron A, Oron U, Streeter J, etal (2007). "low-level laser therapy applied transcranially to mice following traumatic brain injury significantly reduces long-term neurological deficits". J Neurotrauma. 24(4):651-656.
¹⁰Naeser MA, Saltmarche A, Krengel MH, Hamblin MR, Knight JA (2010). "Improved cognitive function after transcranial, light-emitting diode treatments in chronic, traumatic brain injury: two case reports". Photomed Laser Surg. 29(5):351-358.
¹¹Shaw VE, Spana S, Ashkan K, et al (2010). "Neuroprotection of midbrain dopaminergic cells in MPTP-treated mice after near-infrared light treatment". J Comp Neurol. 518(1):25-40.
¹²Michalikova S, Ennaceur A, van Rensburg R, Chazot PL (2008). "Emotional responses and memory performance of middle-aged CD1 mice in a 3D maze: effects of low infrared light". Neurabiol Learn Mem. 89(4):480-488.
¹³Scfiiffer F, Johnston AL, Ravichandran C, et al (2009). "Psychological benefits 2 and 4 weeks after a single treatment with near infrared light to the forehead: a pilot study of 10 patients with major depression and anxiety". Behav Brain Funct. 5:46.
¹⁴Rojas JC, Bruchey KB, Gonzalez-Uma F (2012). "Low-level Light Therapy Improves Cortical Capacity and Memory Retention". J Alzheimers Dis. 2012;32(3):741-52
   Source: RojasJC and Gonzales-Lima F (2011). "Low-level light therapy of the eye and brain". Eye and Brain. 3(64) updated and modified.

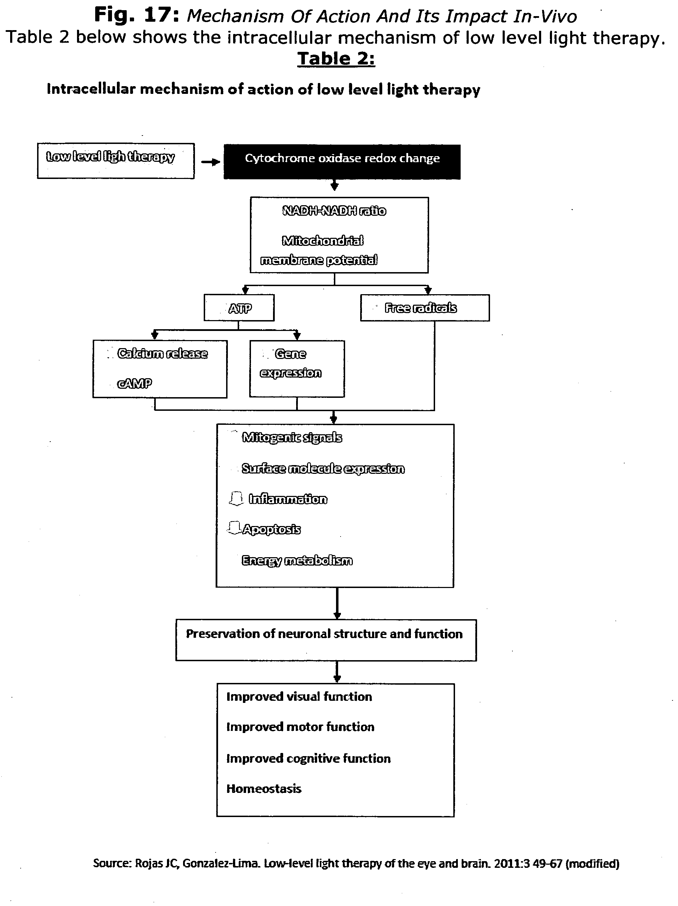

As illustrated by Fig. 17 and the flow chart of Table 2, the key to the therapeutic response of the brain lies in the presence of a photoacceptor respiratory enzyme which exists in all cellular mitochondria, cytochrome oxidase. The cytochrome oxidase enzyme represents the best known intraneural marker of metabolic activity; and its enzyme activity is tightly coupled with free radical metabolism, cell death pathway, and glutamatergic (a neurotransmitter related) activation, important for learning and memory [see for example, Wong-Riley MT (1989). "Cytochrome oxidase: an endogenous metabolic marker for neural activity". Trends Neurosc. 12(3):94-101].

Photoacceptors, unlike photoreceptors found inside the eyes, do not process light energy, but are instead a component part of the normal metabolic pathways. Photoacceptors are sensitive to light in the visible red region and near-infrared region of the light spectrum, and are able to convert the absorbed light of these red and near-infrared wavelengths into cellular energy molecules of adenosine triphosphate (ATP).

When light of these visible red and near-infrared wavelengths (at low energy levels) enter living cells (including nerve cells), the light energy modulates the cell's activity of metabolism (photobiomodulation) by regulating internal mitochondrial function, the intraneuronal signaling systems, and the redox states. Moreover, empirical experiments show that photoneurobiomodulation of electrical activity in neurons can be achieved independently of thermal effects [see Fork RL (1971). "Laser stimulation of nerve cells in Aplysia". Science. 171(974):907-908]. Equally important, when employed and delivered at low energy levels, the therapeutic effects of brain-absorbed light energy are not accompanied by any substantive complications or major side effects. Thus, with the neurons of the brain affecting virtually all functions and activities of the living body, the impact of exposing the brain to modulating light energy consequently affects the entire medical life of the human being.

At the cellular level, the sensitivity of cytochrome oxidase to red light and near infrared red light may be explained by the role of the chromophore in the protein structure. The chromophore is an organic structural entity that is present in all photoreceptors, such as those present in the eyes and which give us the perception of colors. These chromophores will absorb only particular light wavelengths and reject all others; and the cytochrome oxidase in the chromophores are known to accept red and near-infrared red light energy.

These underlying facts accurately identify the potential impact of light energy irradiation that could be purposely directed into one or more anatomic parts of the living brain on-demand, resulting in both beneficial therapy for and prophylaxis against a variety of medically recognized nervous disorders and pathological states.

### Photoacceptors In The Nervous System:

Although earlier-reported animal experiments suggested the presence of photoacceptors in the brain, it was only those particular experiments and empirical results first reported in 2000 which correctly demonstrated that isolated mitochondria are sensitive to irradiation with monochromatic light in the red and near-infrared red regions of the light spectrum. Thus, it was empirically demonstrated that illumination of isolated rat liver mitochondria with red low-powered lasers increased ATP synthesis and oxygen consumption [Karu T (2000). "Mechanism of low-power laser light action on cellular level". Proc SPIE. 2000;4159:1-17].

In addition, it has been empirically demonstrated that impaired mitochondrial oxidative metabolism is associated with neurodegeneration [see Wong-Riley MT et al (2001). "Light emitting diode treatment reverses the effect of TTX on cytochrome oxidase in neurons". Neuroreport. 12(14):3033-3037]. Also, research studies revealed that rat neuronal cultures exposed to low level red light showed increases in cytochrome oxidase activity [see Wong-Riley MT et al (2005). "Photobiomodulation directly benefits primary neurons functionally inactivated by toxins: role of cytochrome c oxidase". J Biol Chem. 280(6):4761-4771].

Accordingly, a light-modulating method and system aimed at improving mitochondrial metabolism in-vivo is deemed to be of major benefit to the functionality of both the diseased and normal brain tissues. Such a light-modulating methodology is also believed to be able to relieve pain in humans [see Chow Rt et al (2009). "Efficacy of low-level laser therapy in the management of neck pain: a systematic review and meta-analysis of randomised placebo or active treatment controlled trials". Lancet. 374(9705): 1897-1908].

It is also noteworthy that the effects of light irradiation on the brain are observed to be effective in a wavelength-specific range. The primary photoacceptor mediating the effects of the light is not only localized to the mitochondria; the molecules that absorb the light in cells are believed to be part of the respiratory chain [see Karu T (1989). "Laser biostimulation: a photobiological phenomenon". J Photochem Photobiol B. 3(4):638-640].

### Equilibrium And Homeostasis:

It is recognized that there comes a point or stage when the photoacceptors (such as cytochrome oxidase) do not respond to further photostimulation. This critical event occurs when the photoacceptors are fully reduced or fully oxidized by the absorbed light energy; thus, photoacceptors can respond to light energy exposure only when they are in their intermediate stage [see Karu TI, et al (2008). "Absorption measurements of cell monolayers relevant to mechanisms of laser phototherapy: reduction or oxidation of cytochrome c oxidase under laser radiation at 632.8 nm". Photomed Laser Surg. 26(6):593-599].

Accordingly, when the photoacceptors become fully reduced or are fully oxidized, further sequential low power irradiation will not yield further metabolic activity from the photoacceptors. This indicates that the living cells in the body have coded action potential limits when they are ex-homeostasis; and thus, the neurons of the brain have the potential to respond positively to light irradiation only until they reach a state of homeostasis.

### III. Neural Conditions Suitable For Light Irradiation Treatment

There are many potential neural conditions that can benefit from overt light irradiation of one or more regions of the brain in-vivo. Some of these medical conditions are summarily described below.

In addition, it will be noted and appreciated that a wide range of other neural diseases, disorders, and pathological states are also envisioned to be effectively therapeutically treatable using the present invention. Examples of these other neural conditions are expected to include: epilepsy, migraine, chronic fatigue syndrome, encephalitis, multiple sclerosis, anxiety disorder, attention deficit disorder, schizophrenia, and learning disabilities.

### Treatment Of Stroke, Neurotrauma, Cognition And An Emotional Mind State:

The human and animal studies that relate to treatment of stroke, neurotrauma, cognition, emotional states, and similar neurological disorders are well documented [see for example, Rojas JC, Gonzalez-Lima F. "Low level light therapy of the eye and brain". Eye and Brain. 2011;3:49-67].

The brain, being the neurological control center of systemic body health, has a direct impact on all body health. For example, the health of the hypothalamus, being the key regulating gland for systemic homeostasis, has a profound impact on overall body health; and thus, a functionally improved hypothalamus will concomitantly yield a greater degree of systemic homeostasis.

Also, research studies have extensively investigated brain irradiation for both stroke and neurotrauma. For example, recent studies by Uozemi *et al.* have demonstrated that low energy light delivered transcranially was able to increase blood flow by 30% [Uozumi Y et al (2010). "Targeted increase in cerebral blood flow by transcranial near-infrared laser irradiation". Lasers SurgMed. 42(6):566-576].

Such demonstrated beneficial results with light irradiation have been accompanied with significant increases in nitric oxide production - a mechanism that is associated with the relaxation of vascular walls to achieve improved blood circulation. Thus, the cerebral blood flow was shown to be increased in both treated and untreated hemispheres. Also, subjects pretreated with light irradiation showed improved blood flow during the period of occlusion, with stable body temperature, heartrate and respiratory rates. The overall result is a significant decrease in apoptotic cells during a stroke event.

Regular irradiation with low level near infrared red (NIR) light has also been found to be associated with significant neurological recovery after stroke events [see Detaboada L et al (2006). "Transcranial application of low-energy laser irradiation improves neurological deficits in rats following acute stroke". Lasers Surg Med. 38(1):70-73]. Furthermore, these recovery
effects were associated with increased neuronal proliferation and migration in the subventricular zone - which plays a role in neurogenesis [see Oron et al (2006). "Low-level laser therapy applied transcranially to rats after induction of stroke significantly reduces long-term neurological deficits". Stroke. 37(10):2620-2624; see also Lampl Y et al (2007). "Infrared laser therapy for ischemic stroke: a new treatment strategy: results of the NeuroThera Effectiveness and Safety Trial-1(NEST-1)". Stroke.38(6):1843-1849].

### Treatment Of Traumatic Brain Injury:

Published research studies have provided in-vivo evidence that the effects of low level light irradiation on cytochrome oxidase and the release of nitric oxide plays a major role in the neuroprotective action of light irradiation therapy not just against ischemia, but also against traumatic brain injury [see Naeser MA et al (2010). "Improved cognitive function after transcranial, light-emitting diode treatments in chronic, traumatic brain injury: two case reports". Photomed Laser Sur. 29(5):351-358].

### Treatment Of Neurodegenerative Diseases:

Light irradiation of the brain has been found to support neurogeneration in-vivo. Thus, light energy irradiation can therapeutically treat a range of different neurodegenerative diseases and disorders - such as Parkinson's disease which is specific to the substantia nigra, a part of the mid-brain area located behind the hypothalamus; and which can be reached with NIR light wavelengths.

In evidence of these beneficial therapeutic effects: In a study using small animals like mice, it was demonstrated that low level light irradiation at 670 nm wavelength helps prevent the loss of dopaminergic cells in the substantia nigra [see Shaw VE et al (2010). "Neuroprotection of midbrain dopaminergic cells in MPTP-treated mice after near-infrared light treatment". J Comp Neurol. 518(1):25-40].

However, longer wavelengths of light energy [such as near-infrared light (NIR)] are deemed to be more feasible for much larger mammalian subjects such as a human being.

### Treatment Of Dementia And Alzheimer's Disease:

Neurodegeneration can lead to cognitive impairment that is often medically identified with dementia. Accordingly, causing an improved blood flow demonstrates the true therapeutic potential for addressing and treating vascular dementia.

Alzheimer's disease, although medically a form of dementia, apparently has a variety of different causes. The early signs/symptoms of this neurodegenerative condition are typically revealed as regional brain metabolic deficits in the form of reduced cytochrome oxidase activity - an overt sign for potential risk of Alzheimer's disease [see Valla J et al (2001). "Energy hypometabolism in posterior cingulated cortex of Alzheimer's patients: superficial laminar cytochrome oxidase associated with disease duration". J Neurosci. 21(13):4923-4930].

Because brain irradiation with red and infrared red light energy demonstrably activates cytochrome oxidase, a light irradiation treatment procedure can help manage the symptomatic onset of a full Alzheimer's disease state.

### Treatment Of Depression And Similar Emotional Deficits:

Phenotypic expressions of mood disorders such as depression and post-traumatic stress disorder (PTSD) have been shown to be associated with decreased metabolic capacity in the prefrontal cortex region [see Shumake J, Gonzalez-Lima F (2003). "Brain systems underlying susceptibility to helplessness and depression". Behav Cogn Neurosci Rev. 2(3):198-221]. Electrical stimulation of the prefrontal cortex has antidepressant effects [Hamani C et al (2010). "Antidepressant-like effects of medial prefrontal cortex deep brain stimulation in rats". Biol Psychiatry. 67(2):117-124].

Thus, light irradiation of the prefrontal cortex region with red light and near-infrared red light should cause an increase of metabolic capacity in the prefrontal cortex region, as well as provide potential neuroprotection against these medical conditions. Indeed, a pilot study showed that when the foreheads of human patients suffering from major depression and anxiety were light irradiated with low level light with 810 nm wavelength, the blood flow to the frontal cortex increased and induced a 63% reduction in depression scores [see Schiffer F (2009). "Psychological benefits 2 and 4 weeks after a single treatment with near infrared light to the forehead: a pilot studyof 10 patients with major depression and anxiety". Behav Brain Funct. 5:46].

### Treatment Of Memory Deficits:

Research studies have demonstrated that irradiation of the prefrontal cortex region of the brain with near-infrared red light of 1072 nm wavelength improved the individual's functional memory [see Mikhalikova S et al (2008). "Emotional responses and memory performance of middle-age CD1 mice in a 3D maze: effects of low infrared light". Neurobiol Learn Mem. 89(4):480-488].

As this memory deficit condition is common among the more elderly, using light irradiation method to treat the prefrontal cortex region of the brain can help with the aging-related problem of working memory deficits.

### IV. The System Of The Present Invention

### Choices Of Method/System Specifications And Dosimetry:

An effective and safe light irradiation method and system in compliance with the present invention provides choices and control over certain operational parameters. These operational parameters include the choice(s) of: the light wavelength, coherency or non-coherency, energy [as measured in Joules (J)], Power [as measured in Watts (W) or milliwatts (mw)], irradiance (W/cm²), radiant exposure (J/cm²), exposure time (seconds), wave type (continuous or pulsed), fraction protocol (number of patient treatment sessions), light beam size (area of landed beam), and light beam penetration (delivery) distance.

### 1. Choice Of Therapeutic Wavelengths

The wavelengths shown to be most effective at inducing in vivo beneficial effects in living neural cells have been in the optical window of the red and near-infrared red range (NIR) of the spectrum - *i.e.,* between 600 nm and 1100 nm wavelengths. Successful treatments for brain irradiation have typically been performed at 633-670 nm (visible red) wavelengths or 808-1072 nm (near-infrared) wavelengths in both animals and humans. Accordingly, any light wavelength ranging between about 600 nm and 1100 nm is deemed to be acceptable for therapeutic use with the present invention.

In general however, the longer the wavelength of light, the lower the energy required for successful treatment; and it is well established that the longer the light wavelength, the deeper the penetration distance of the light passing into and through living tissues.

In the present method and system of brain irradiation through the nasal cavity, the shorter visible red light wavelengths between about 600 nm-690 nm are able to perform as well as the near-infrared (NIR) wavelengths between about 700 nm-1400 nm for certain conditions.

Great importance is given to the fact that it is these wide ranges of light wavelength induce the greatest mitochondrial response, as opposed to the need to have one particular wavelength for effective tissue penetration distance in-vivo. Furthermore, light photons wavelengths between 630 nm and 800 nm will penetrate living tissues and travel up to 28 mm distance even through layers with relatively low transparencies such as skin, connective tissue, muscle, bone, and spinal cord - with about 6% of the total energy density being detectable. Therefore, should depth of penetration be a critical factor for the medical condition or pathological state being treated, the NIR light wavelengths between about 700 nm-1400 nm are preferred for use based on the fact that the longer wavelengths penetrate deeper into the tissues.

Note also that penetration of light energy through living tissues depends not only on the chosen wavelength but also on the optical properties of the targeted tissues. In particular, the maximal penetration distance of light energy within the gray and white matter of the brain occurs at wavelengths between about 700 nm-1400 nm in the NIR light region. For this reason also, the NIR light wavelengths between about 700 nm-1400 nm are highly preferred for use.

It is also generally preferable to select and use a single monochromatic wavelength of light for a single therapeutic application. Typically therefore, the single monochromatic wavelength chosen should be about 670 nm (visible red) or about 830 nm (near-infrared red).

Moreover, simultaneous dichromatic irradiation changes the ratio of the reduced and oxidized form of the enzymes; thus, it is recommended that the user select pure monochromatic wavelength light source for almost every therapeutic application. As a guide to selecting specific therapeutic wavelengths of light, it is suggested that light wavelengths in the region of 633 to 670 nm be employed for general brain irradiation; and that light wavelengths of about 808 to 1072 nm be used to penetrate and reach the deeper anatomic regions of the brain.

### 2. Choice Of Coherent vs Non-Coherent Radiation (lasers vs light-emitting diodes)

Lasers provide coherent electromagnetic radiation that is unidirectional, hence allowing for a more concentrated energy coupled with a high energy input. Also modern laser light sources are usually constructed in low intensity semiconductor formats, with a built-in divergence that allow for a high degree of safety (often about 57 degrees divergence).

Such laser light sources have distinct advantages which include: A higher degree of tissue penetration; an efficient optic coupling; and a high monochromaticity. When a deeper penetration distance of living tissues is required [given the same parameters of wavelength, energy dosage and intensity], the coherent ligh of lasers is more desirable than the non-coherent light generated by light-emitting diodes (LEDs).

However, for most therapeutic applications, light coherency as such is not required for clinical efficacy; and in those medical circumstances where a greater distance of tissue penetration is needed, it is deemed better met using non-coherent light at longer wavelengths from light-emitting diodes (LEDs).

Consequently in recent years, light-emitting diodes (LEDs) have become viable therapeutic alternatives to lasers as light sources. It is postulated that the cell's photoacceptors (particularly cytochrome oxidase) do not discern between the coherency or non-coherency of the light photons that are received. Therefore, given the same wavelength of light, the energy dosage and intensity input received at the cell's photoacceptor receptors using light-emitting diodes (LEDs) will yield therapeutic outcomes which are very similar or identical to that provided by coherent light of laser light sources. Although penetration with LED non-coherent light is typically shallower, the LED generated non-coherent light has the advantage of providing a wider area of irradiation beam coverage.

The system of the present invention recognizes the coherent vs. non-coherent differences existing between light from laser sources and light-emitting diode sources; and provides for both possibilities by carefully choosing between them on the basis of the optimum condition for particular application purposes - *i.e.,* the particular disease state or disorder to be therapeutically treated will dictate which is the better format.

Therefore as merely a first illustrative example, when there is an advantage in irradiating only one specified area in the mid-brain area - such as irradiating the more deeply located pineal gland in order to restore normal circadian rhythms and correct sleep disorders, the coherent light of the laser light source is generally preferred for its greater tissue penetration distance. As a meaningful alternative however, the use of non-coherent LED light at a longer wavelength (preferably in the NIR range) in combination with a longer treatment time will adequately compensate for the loss of maximal tissue penetration distance that can be provided by the coherent light of the laser source.

As a second illustrative example, whereas the coherent light from a NIR 810 nm laser source would be most favored because of its deeper tissue penetration capabilities, the 810 nm laser light itself is invisible to the human eye. Thus, the user of the instant method and system has no visible light as such to trigger eye blinking as an autonomic defense mechanism to accidental eye exposure; and the user runs a substantial risk of inadvertently causing a major retinal injury to the eye if he is careless. Hence the system of the present invention offers a guided approach which recommends that when the 810 nm light wavelength is employed and is intended for unsupervised personal therapeutic usage at home - such 810 nm light should be made available to the purchasing public only in the LED light source version alone. Similarly, the laser sources of
the 810 nm light wavelength should be reserved and limited for therapeutic use solely within the research domain or in a supervised medical treatment environment. Alternatively, it is suggested that laser sources be used to generate visible red light at the 655 nm wavelength in order to benefit from the safety aspect of having a visible red light, as well as concomitantly to provide the greater tissue penetrative advantages of laser light.

Another important aspect of non-coherent LED generated light is that the use of such non-coherent light creates a very negligible amount of heat in comparison to laser generated light. This valuable feature of non-coherent LED generated light allows the living brain tissue to be exposed for longer periods of time using wavelengths at relatively low power densities, which in turn allows for more efficacious modulation of neural metabolism. Thus, if the treatment time is to be prolonged for medical efficacy - as exemplified by the treatment of traumatic brain injury, non-coherent LED generated light wavelengths at relatively low power densities are highly favored over the use of laser generated light in order to avoid the risk of causing undesired thermal injury to the brain tissue.

For general therapeutic use purposes therefore, the present invention favors the use of LED light sources and non-coherent light wavelengths (especially in the NIR wavelength range owing to its greater tissue penetrative quality) for therapy treatments as well as for preventive medicine applications. This favored recommendation generally includes and encompasses those medical/clinical/pathological conditions relating to human cognitive functions, neurodegeneration, vascular dementia, migraine, pain, and human memory deficits.

In comparison, given the same wavelengths, low power level coherent light from laser sources is preferable for treating acute and chronic neurological disorders and conditions, and is desirable for treating specifically targeted regions/areas which are more deeply anatomically located within the interior of the brain. Thus, visible red light irradiation treatment of Parkinson's disease (involving the substantia nigra), sleep disorders related to the circadian rhythm (pineal gland), and accelerated rehabilitation (hypothalamus) are better treated using low level coherent light from lasers when compared to LED of the same wavelength. Also for safety reasons, only visible red light wavelengths between 600 nm to 690 nm should be used with minimal safety restrictions.

### 3. Therapeutic Energy And Other Parameters

Light energy is traditionally measured as Joules (J) = Power (W) X Time (seconds). For brain stimulation purposes, very little light energy is required to stimulate mitochondrial activity, although a definitive minimal threshold amount for medical efficacy is yet to be established as such.

Instead, the proper reference point for medical efficacy in use today is the time-tested intravenous light irradiation technique involving light being directly injected into the vein (used mainly in Russia, Germany and many other countries around the world for decades), and which normally follow the parameters of lasers with a wavelength of 632.8 nm, a power of 1.5 mw, and a time of 30 minutes per treatment session. On this basis, patients are usually treated once a day for the first three calendar days, and then treated once every two calendar days - until a total of ten patient treatment sessions is reached. For each patient treatment session, light energy of 2.7 Joules (1.5/1000W X 30 minutes X 60 seconds) is delivered.

When brain irradiation with laser generated light (identical or similar to the 632.8 nm wavelength) is applied from within the nasal cavity, there are adjacent tissue depth issues to consider. These issues are overcome by increasing the Power [measured in Watts (W) or milliwatts (mw)] by several orders of magnitude, such as 5 mw for 25 minutes, thereby generating an energy output of 7.5 Joules (5/1000W x 25 minutes X 60 seconds).

Also, adding greater Power to a pulsed light source delivers more energy, which in turn can activate more ATP in-situ; however, such added Power to the system compromises the usability of the methodology in the intranasal embodiment.

A reference Power parameter for laser embodiments of the invention is to limit the Power factor to 5 mw in order to keep the Power at the low risk level of Class 3R [a standard set by the US Food and Drug Authority (FDA)]. Thus, a preferred set of operational parameters for the laser embodiments would offer a Power factor of 5 mw and a time treatment duration limited to 25 minutes for each patient treatment session. In this manner, the laser generated light will pass through the underlying subcutaneous layers of tissue adjacent to the nasal cavity and enter into the brain efficaciously.

In comparison, it is noted that the LED light source is mostly divergent. Accordingly, when using the LED embodiments of the invention, the energy output is to be increased by about 60% to about 12 Joules. This change in energy output also calls for a higher Power factor now set at 8 mw (12 Joules /(25 X 60 seconds) X 1000).

In summary therefore, for a laser light source, the energy aimed for is about 7.5 Joules (5/1000W X 25 X 60 seconds); and for a LED light source, the energy aimed for is about 12 Joules (8/1000WX 25 X 60 seconds).

### 4. Continuous Wave (CW) vs Pulsed Frequency

The published scientific literature has factually established that when using 808 nm laser light, pulsed laser beams at 100 Hz and 1000 Hz produce superior results to continuous wave (CW) beams at 100 Hz and 1000 Hz; and that a 810 nm laser beam pulsed at 10 Hz produced a greater clinical recovery from traumatic brain injury than a continuous wave beam at 100 Hz.

Why pulsing at 10 Hz works better than 100 Hz (or any other frequency) remains and is a matter of speculation: It is noted that 10 Hz is the frequency of alpha brain waves; and thus pulsation at 10 Hz resonates with the whole brain at rest. Furthermore, the hippocampus region of the brain also functions at waves in the 4-10 Hz range. The hippocampus is responsible for memory, emotional well-being, behavioral management, spatial memory and navigation. It also is one of the regions that suffer severe damage in advanced Alzheimer's disease.

In summary, for brain therapy using the present method and system, and particularly for treatments of the mid-brain region, a 10 Hz pulsed model is most preferred for efficacious clinical outcomes - especially when combined with a NIR 810 nm light source, which gives good penetration depth.

### Invention Operational Parameters Overall:

A. Based on the operational descriptive details given above, the operational parameters for intranasal light irradiation and brain stimulation may be broadly divided into three Indications sub-groupings that embrace a wide range and variety of clinical applications, and which are presented by Table 3 below.

**Table 3:**

| Invention parameters | | | |
|---|---|---|---|
| | Indications | | |
| | Embodiment 1 | Embodiment 2 | Embodiment 3 |
| Parameter | For general well-being and non-specific healing, including (general guide only): | For more specific, acute and chronic disorder, particularly mid-brain related, including (general guide only): | For deeper penetration and wider irradiation; covers most areas of the brain (general guide only): |
| | Preventive medicine, systemic homeostasis, facial area and systemic pain (eg fibromyalgia), cognitive performance, working memory, age-related neurodegeneration chronic fatigue, early dementia, depression, PTSD. | Acute and chronic neurological disorders, muscular recovery, anxiety disorder, sleep disorder, acute fatigue, depression, emotional disorder, memory deficiency | Covers the indications of the other 2 embodiments, and including deeper lying dysfunctional sources, targeting Parkinson's Disease, Alzheimer's Disease etc. |
| Wavelength | 633 nm (visible red) | 655 nm (visible red) | 810 nm (near-infrared) |
| Mode | LED | Laser | LED |
| Power | 6-8 mw | 5 mw | 6-8 mw |
| Single session duration | 25 minutes | 25 minutes | 25 minutes |
| Wave/pulse operation | Continuous wave | Continuous wave | 10 Hz pulse |
| Duty cycle | Not applicable | 50% | 50% |
| Energy delivery | 11-13 J/cm2 | 8 J/cm2 | 11-13 J/cm2 |
| Direction | Angled to cover general brain area | Angled mainly at mid-biain area | Angled to cover general brain area |

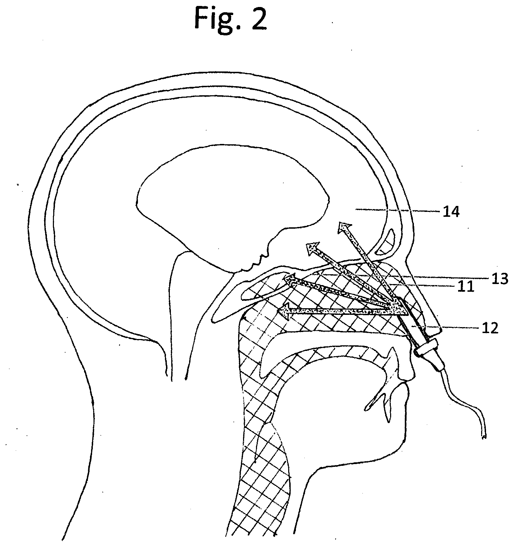

B. In addition, conceptual representations of how the three systems target the human brain are shown by Figs. 2, 3 and 4 respectively, and can be summarized as follows:

Fig. 2 illustrates a model system using a 633 nm LED source: The light rays 11 from the LED light source 12 are generally unimpeded and dispersed over a wide area until they reach the perpendicular plate of the ethmoid bone 13. The penetration into the brain is relatively shallow but the extensive neural network distributes the signal throughout the brain. It targets the prefrontal cortex 14 first and then the brain in general. However, the light source can be angled to point at any region of the brain as desired.

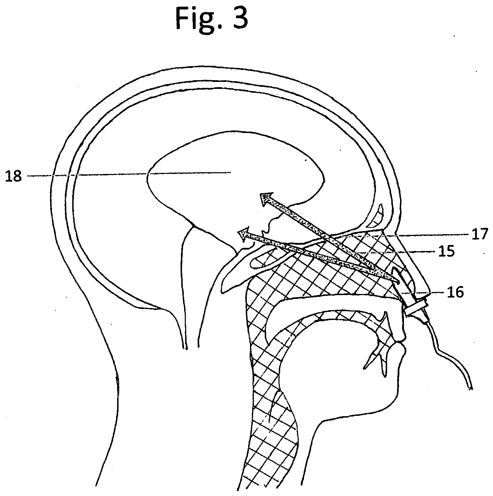

Fig. 3 illustrates a model system using a 655 nm Laser source: The light rays 15 from the laser light source 16 are generally unimpeded and generally stay coherent within a narrow dispersion until they penetrate the perpendicular plate of the ethmoid bone 17. The penetration is deeper relative to the 633 nm LED model in Fig. 2 and reaches the main targeted mid-brain region 18. However, secondary signaling will still be distributed throughout the other brain regions.

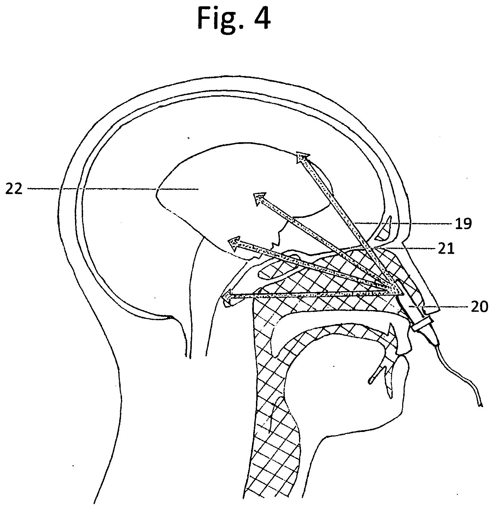

Fig. 4 illustrates a model system using 810 nm LED light source: The light rays 19 from the LED light source 20 are generally unimpeded and dispersed over a wide area until they reach the perpendicular plate of the ethmoid bone 21. The penetration is deeper relative to the 633 nm LED model in Fig. 2 and the dispersion (and tissue coverage) is wider than the 655 nm laser in Fig. 3, covering the whole brain, including the deeper lying mid-brain area 22. This model system extracts the good feature of the other two models described above. The commercial disadvantage is that the light is not visible and some users may be more comfortable and safe with light rays they can see.

### V. Preferred Structural Embodiments Of The Invention

It will be recognized and appreciated that the present invention is a Continuation-In-Part of U.S. Non-Provisional Patent Application Serial No. 12/802,866 filed June 16th, 2010 and entitled "Method And Portable System For Non-Invasive, In-Vivo Blood Irradiation Light Therapy", now pending. Thus if further description and/or more extensive details concerning the apparatus of the present invention are desired, U.S. Non-Provisional Patent Application Serial No. 12/802,866 can and will provide them.

### Preferred Apparatus And System Embodiments:

The various model apparatuses and systems of the present invention employ a common user interface for a consistent image and economy. This user interface embodiment may be improved or modified over time; but in each embodiment the software and structural components will determine the manner in which the light wavelengths and the energy intensity are effectively delivered to the brain.

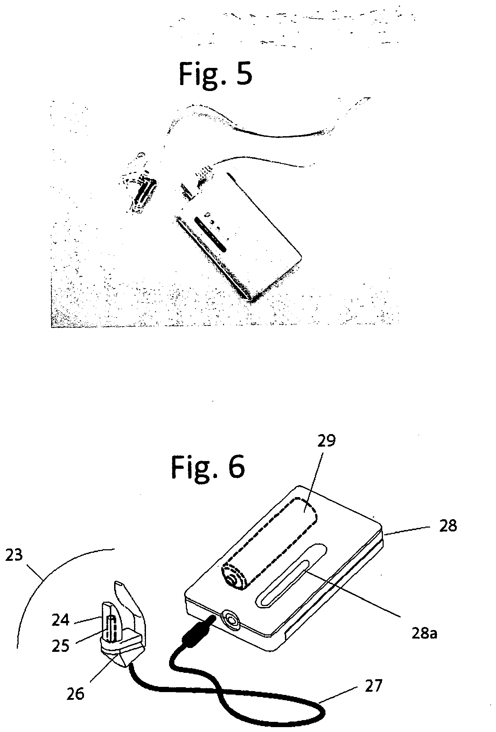

Clearly, it is intended that a preferred embodiment of the user interface be one which can be manufactured as a commercially saleable product, and that commercial format is presently considered to be the best mode of the invention. Accordingly, this best mode of the non-invasive self-administrable apparatus is shown by Figs. 5-12 and Figs. 15-18 respectively as a unified system and ready to use medical device.

As seen therein, the non-invasive apparatus Figs. 5 & 6 provides an self-administrable applicator device 23, a structural article of convenience, which holds and supports a configured irradiation lens 24 in a desired fixed position within the nasal cavity; and which is dimensionally small in size, is at least partially transparent, and is purposefully shaped to allow its insertion into a nasal cavity space without causing meaningful impairment to the subject's ability to breathe.

The self-administrable applicator device 23 is formed as a resulting combination and integration of two separate structural entities: a transparent and partially hollow configured irradiation lens 24 having at least one discrete light diode within its housing 25; and a support base 26 that connects the configured irradiation lens 24 with a connecting power cable 27.

Structurally, the configured irradiation lens 24 encapsulates a diode housing 25 which contains the diode light source in a form either as a light emitting diode (LED) or as a laser. In addition, the configured irradiation lens 24 is formed at least in part of a light transmitting material; and together with the diode housing 25, is angled and integrated with the lens to release the light photons at any desired direction and dispersion angles.

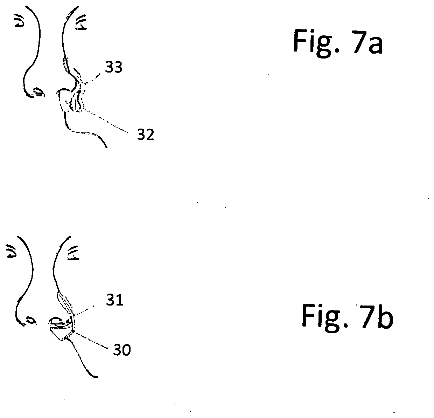

In the embodiment of the self-administrable applicator means shown by Figs. 5-7, the device can include one or more support structures formed of different plastics that serve to hold the configured irradiation lens 24 - i.e., the lens/diode complex - rigidly in the desired direction, and yet hold the configured irradiation lens 24 securely to the anatomy of the nose comfortably. The applicator device may be in a set format as seen in Fig. 7a; or can be manipulated with a cantilever as shown by Fig. 7b.

In the more desired embodiments, the system specifications are controlled by a circuit board containing an embedded software program(s) that is housed in a controller unit 28, and which is powered by a disposable dry cell battery. In the preferred embodiment and the set format of the applicator shown by Fig. 7b, the support structures 30 are rigid and set, whereas the clip 31 is flexible. Both the support structures 30 and the clip 31 are molded out of durable plastic materials.

In the embodiment having the cantilever-based applicator device as shown by Fig. 7a, the support base carries a cantilever 32 - which when depressed with the fingers, will open and then allow the applicator to slide the lens into the cavity space of the nostril. The clip 33 will hold the applicator device securely to the nose.

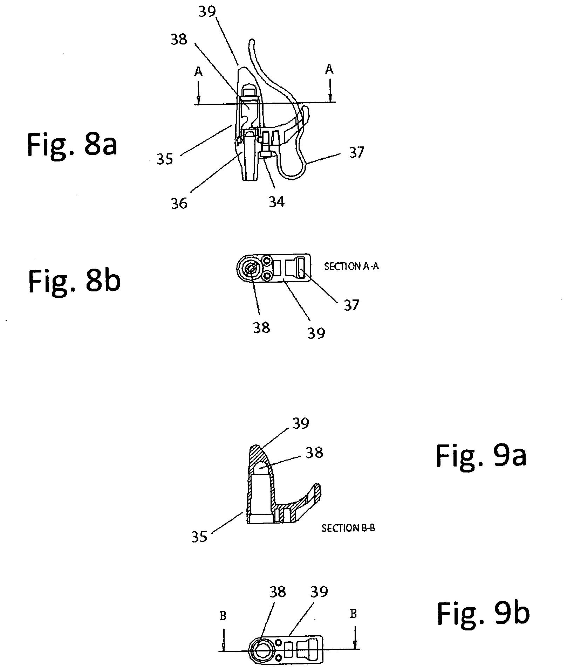

In the preferred format illustrated by Figs. 8a, 8b, 9a, and 9b respectively, the applicator device is a single cooperative entity which includes a support base 34 for the configured irradiation lens 35; and is collectively structured as a discrete cradle section 36 and a contoured nose clip or fitting 37 fashioned for easy at will attachment to and detachment from the exterior surface of the human nostril. The configured irradiation lens is part of a single clear plastic molding that dimensionally extends to encase part of the support base 39. The nose clip 37 - apart from serving physically as contoured friction fitting by which to hold the configured irradiation lens 35 in proper position within the nostril - is preferably composed of a white or opaque material which contributes to its service as a light barrier and reflector; and which acts to redirect substantial stray light passing through the tissue wall of the nostril back towards the interior of the nasal cavity wall.

In addition, as shown by Figs. 5-6 respectively, the self-administrable applicator means 23 is in electrical communication (via a cable and jack module connector 27) with a process controller assembly 28 - a control and power supply construct which is compact, lightweight and sufficiently portable to be carried by hand, fit inside a shirt pocket, or be clipped to a shirt. In this highly preferred embodiment, the process controller assembly 28 includes a portable and disposable/replenishable source of on-demand direct electrical current; and is able to convey carefully regulated dosages of electric power on-demand to the light generating unit(s) 29 contained within with the applicator assembly 23 for light irradiation therapy. In this instance, the process and power controller assembly 28 also includes and provides an automatic timer and power switch 28a. The controller automatically shuts off the electric current conveyed to the light generating unit(s) after the passing of a prechosen amount of time.

### The configured irradiation lens

Architecturally as shown in detail by Figs. 8 and 9 respectively, the preferred configured irradiation lens 39 appears as a substantially "L" shaped construct. However, the configured irradiation lens 39 as such is always formed by and is the result of combining and integrating two other separate and essential structural entities:
(i) A portable hollow casing 35, formed in least in part of a light transmitting material, and which serves at least in part as a reflective lens that reflects light in a desired direction; and
(ii) At least one discrete light generating unit or diode 38, which is entirely housed and contained within the interior spatial volume of the hollow casing 35.

Together the discrete light generating unit 38 and the portable hollow casing 35 collectively form the configured irradiation lens 39, a construct able to emit and direct light energy of at least one predetermined wavelength, power and pulsed (or continuous wave) mode on-demand. Thus, in this illustrated embodiment and use context, the configured irradiation lens 39 as a whole includes the entire "L" shaped construction.

It is critical, therefore to understand and appreciate the meaning and effect of this "L" shaped construction; and particular attention is therefore directed to the views provided by Figs. 8a-8b and Figs. 9a-9b respectively.

As shown therein, although the hollow casing 35 is formed as a lens and includes the entire "L" shaped structure 39, the light generating unit 38 is typically placed into and contained by only the vertical or upright volumetric portion of the "L" shaped casing 39.

Consequently via this arrangement illustrated by Figs. 8a, 8b, 9a, and 9b respectively, the horizontal or axial portion 39 is typically devoid of any internal contents; and exists merely for physical support by the cradle section 36 of the supporting base 34 in the applicator.

Also, via this positioning arrangement, it is only the vertical or upright volumetric portion 35 of the "L" shaped casing which must be formed of a light transmitting or transparent material. In contrast, the horizontal or axial portion of the hollow casing 39 may be formed of any resilient material, transparent or not.

### The clip as a reflecting accessory and its function

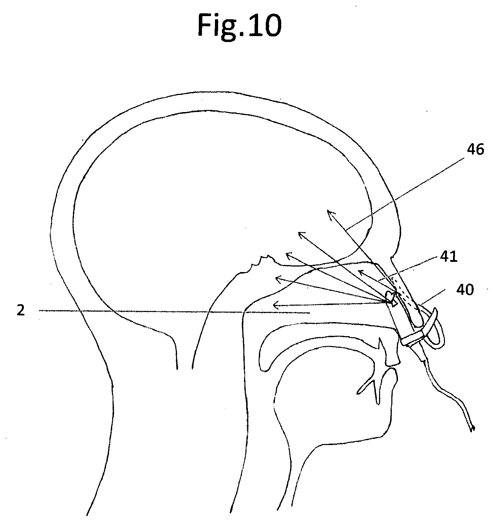

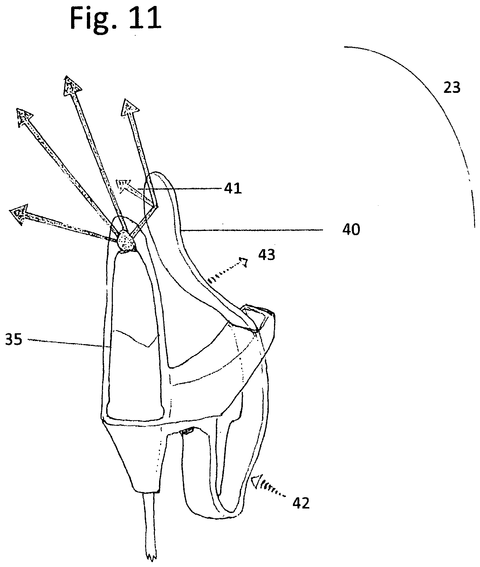

In the preferred embodiments illustrated by Figs. 10 and 11 respectively, the nose clip 40 doubles as a functioning clip by which to secure the applicator device to the nose anatomy; and to function as an accessory to minimize leakage of photons; as well as to reflect these photons 41 back into the nasal cavity to maximize power efficiency. The applicator article is molded with a cantilever which functions by depressing the bottom 42 (with a finger) to allow the clip to open 43 the lens assembly 35 and to slip it comfortably onto the nose comfortably. Once the finger pressure is released, the clip will resume its prior default position and thereby secure the applicator device to the structural nose anatomy.

### The micro-lens and other light-directing configurations

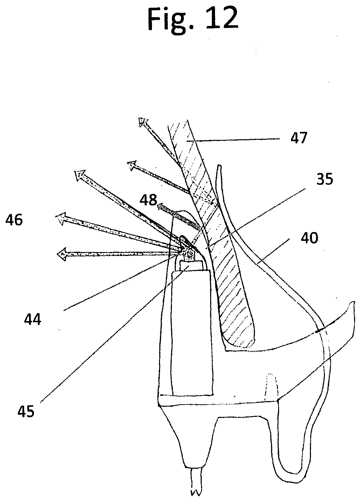

Fig 12 is an illustration showing a low level laser embodiment with the option of a micro-lens 44 which serves to deflect the light released by the diode 45 towards the chosen target area(s). By incorporating this feature, the embodiment minimizes the dispersion of light 46, and minimizes the power requirement to achieve efficacy. Also, much of the remaining leakage is captured and reflected back by nose clip 40. All this occurs while the apparatus as a whole is attached securely to the nose 47.

The micro-lens structure may be designed differently to cater for different wavelengths, and to cater for a coherency factor of the light. Accordingly, the micro-lens configuration can alternatively be a reverse tear drop shape; or an oval; or an oblong shape; or any other rotund configuration - as well as be of any dimensional size which redirects light particles towards a desired direction.

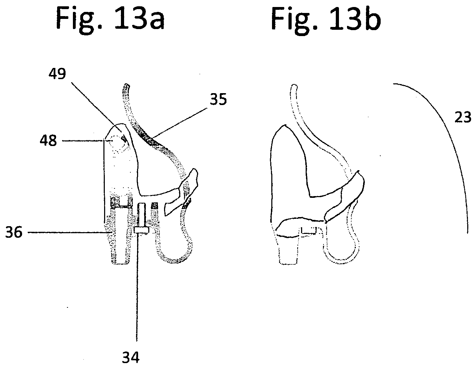

Figs. 13a and 13b show views of an LED embodiment which depends on the mounting angle of the LED diode 48 and a side support 49 to provide the primary manipulation of the light direction. In addition the above format factors, the transparent hollow casing 35 is also configured to deflect the light back into the nasal cavity 48.

In summary, several format factors contribute to directing the generated light into the nasal cavity space and then towards the targeted areas of the brain. These include: the micro-lens 44 for the low level laser embodiment; the mounting angle for the LED embodiment 48; the hollow lens casing 35; and the nose clip 40. In this manner, the amount of electrical energy needed to achieve therapeutic efficacy is kept to a minimum, and achieves the desirable goal of the apparatus being small, portable and convenient to use.

### The self-administrable applicator means

The applicator means 23 as presented by Figs. 6-13 respectively is a purposeful grouping together of parts structurally designed to form a desirable, hand-held article which can be manipulated by the patient with his fingers and is suitable for at will attachment to and detachment from a nostril. As shown by Figs. 6a -6b and 13a - 13b in particular, it is the combination of the configured irradiation lens 39 together with the support base 34 and the nose clip 35 or 40 which collectively form and constitute the applicator means 23; and as such, the applicator means 23 is a self-administrable construction of personal convenience for the user.

It will be therefore appreciated that it is the configured irradiation lens assembly 39 which is the truly essential component - a distinct entity which is desirably held within and is supported by a cradle 36 in a supporting base 34. The cradle 36 holds and aligns the configured irradiation lens 39 for easy and rapid insertion into the nasal cavity space.

Note also that the styled nose clip 40 of the applicator 23 is a structural material arm and outward extension of the supporting base 34. Typically, the nose clip 40 is formed of a white or opaque material which is both flexible and resilient. Two different structural formats of the nose clip are shown by Figs. 7a, 7b, 8a, 8b, 9a, and 9b respectively.

In its preferred embodiments, the nose clip serves two different purposes and functions as represented by Fig. 11: First, it is employed for direct pressure contact against and fitted frictional engagement with the exterior surface of the human nose. This engagement maintains the inserted irradiation lens in proper position within the nasal cavity space. Second, the white or opaque material of the nose clip will reduce leakage of some light particles and reflect stray light particles back into the interior tissues of the nostril. The nose clip 40 performs both of these intended functions as a merged part of the applicator 23. For these reasons, the applicator 23 as a distinct entity - and in contrast to the configured irradiation lens 39 - is merely article of convenience which facilitates usage of the apparatus as a whole.

The applicator means 23 is easy to manipulate using the fingers of the human hand; and thus is a very desirable vehicle for the proper positioning of the portable casing 28 and the light generating unit 45 adjacent to the internal lining of a subject's nasal cavity in the manner shown by Fig. 12. In addition, the interchangeability of the applicator means that houses the light generator unit allows for different users in a family to have their personal applicators (for hygiene reasons) and to share a single controller and thus save the cost of having separate controllers.

Nevertheless, the self-administrable applicator means 23 shown by Figs. 6-12 respectively is deemed to be only one preferred exemplary instance and tangible means for supporting and properly placing the configured irradiation lens 39 within a nostril; and represents only one kind of support construct which makes the in-vivo placement of the configured irradiation lens 39 within a nostril cavity easier, faster and simpler.

### VI. The Essential Components Of The Apparatus

Accordingly, as revealed by the description presented above, it is clear that the non-invasive apparatus of the present invention has four essential component parts. These are:
(i) a portable hollow casing, which also serves as a reflective lens;
(ii) a discrete light generating unit
   which is housed and contained within the interior spatial volume of the hollow casing;
(iii) an identifiable source of electrical current; and
(iv) a processing and power controller assembly.

These four requisite components are electrically linked together by: at least one connector in electrical communication with the source of electrical current for on-demand transfer of direct electrical current to the controller assembly; and at least one connector in electrical communication with the controller assembly and the light generating unit for on-demand conveyance of direct electrical current from the controller assembly. Collectively, the components fulfill the aim of the invention to have the apparatus giving the user full mobility while delivering light of chosen specification to the desired areas of the brain, whether targeted or untargeted.

### Component Particulars:

Particulars of the structural details and attributes for each of the four essential component parts are illustrated by Figs. 6-12 respectively; and a more detailed description of each requisite component part is presented below.

### 1. A Portable Hollow Casing

Each embodiment of the instant invention will include a portable hollow casing having fixed dimensions; a sized internal spatial volume; and an external surface configuration suitable for in-vivo insertion into the nasal cavity space of a nostril, without causing substantial impairment to the subject's ability to breathe.

Typically, the portable casing will be constructed and formed of a light transmitting material over at least a portion of its external surface, and will encompass that volumetric zone intended for housing and containment of at least one light generating unit. By definition, such light transmitting material includes and encompasses transparent, translucent and opaque matter.
However, in most instances, a completely clear and transparent matter is deemed best for use.

It is also important to note that the intended purposes and goals of the portable casing are twofold: (1) to serve as a containment chamber that is configured for easy in-vivo insertion into the nasal cavity space of a nostril; and (2) to act as a molded lens that reflects and directs emitted light waves and particles into the deeper regions of the nasal cavity walls for irradiation
of the circulating blood.

Consequently, the portable hollow casing must have dimensions which are small enough to allow insertion into one nostril; and will minimize impairment of the subject's ability to breathe; and yet will be able to maximize the scattering of the light particles towards the walls of the subject's nasal cavity.

For these reasons, it is very desirable that the hollow casing be fashioned in size and configuration for support by a tangible holder or fixture which the human subject can hold with his fingers. Thus, while the portable casing can be fashioned into any generally slender and elongated shape such as a tubular, or cigar-shaped, or cylindrical format - it is deemed both useful and appropriate that the overall configuration of the portable hollow casing also provide a structural means for support which allows its placement into a nasal cavity space at will.

For this reason also, the "L" shaped format illustrated by Fig. 9 is very desirable and is considered to be an optimal configuration.

### 2. The Light Generating Unit(s)

Each light generating unit is capable of generating light energy of at least one preselected wavelength on-demand. It is intended that the light generating unit will be able to deliver therapeutic light at wavelengths that include the following: In the visible color spectral ranges - the visible red light wavelengths ranging between about 620-700 nm; and in the non-visible spectral ranges - the near-infrared light wavelengths ranging between about 700-1100 nm.

In addition, the generated light energy waves and particles may alternatively be either coherent (as in lasers) or incoherent; be either pulsed or non-pulsed (continuous wave) in delivery; be either constant or non-constant in intensity; be either uniform or non-uniform in phase; polarized and non-polarized; and have a regular or irregular flux.

Any conventionally known means for generating electromagnetic radiation or articles for propagating radiant energy are acceptable for use in the present apparatus; and in the majority of embodiments, it is intended and expected that either a low level laser unit or a light emitting diode (LED) will be employed as the light generating unit(s) for irradiating purposes.

Accordingly, the apparatus as a whole requires only a functional light generating unit or units; and it is of no consequence to the present invention what the nature, or construction, or format of the light generating unit might be so long as it generates and transmits light of at least one prechosen and therapeutically effective wavelength.

### 3. Therapeutically Useful Light Wavelengths

The preferred embodiments would use light at visible red and near- infrared red ("NIR") wavelengths of the light spectrum - *i.e.,* between about 620 nm to 1400 nm. This does not preclude the possibility of deploying light in other wavelengths from ultraviolet B (about 280 nm) to the visible red wavelength region (to 620 nm).

For completeness, if and when desired or needed, the entire spectrum of visible and invisible light wavelengths that can be provided by one or more of the light generating unit(s) of the apparatus. The spectrum of visible and invisible light wavelengths that can be generated on demand by the apparatus if desired is identified by Table 4 below.

**Table 4: Visible and near-visible Color Wavelength Ranges**

| | |
|---|---|
| Ultraviolet A&B: | 280 - 400nm |
| Violet: | 400 - 420 nm |
| Indigo: | 420 - 440 nm |
| Blue: | 440 - 490 nm |
| Green: | 490 - 570 nm |
| Yellow: | 570 - 585 nm |
| Orange: | 585 - 620 nm |
| Red: | 620 - 780 nm |
| Near-Infrared Red: | 780 - 1400 nm |

### 4. Therapeutically Effective Light Ranges And Wavelengths

A guiding principle of the invention is to transmit and deliver a therapeutically efficacious energy for the neurosystem. For this purpose, it is generally preferred that at least coherent visible red light generated by a low level laser, or non-coherent visible red light generated by a LED, and is fixed at wavelengths ranging between 620-1400 nm be used.

For these reasons, various preferred embodiments of the apparatus and system will transmit and direct either coherent or incoherent visible light energy at red color wavelengths ranging between 620-1400 nm, with a radiant power between 1-20 mW. In comparison, some embodiments of the irradiation apparatus and system will emit and deliver visible light energy at wavelengths of between 495-570 nm with a radiant power of 1-20 mW (the green color range); and, in the alternative, still other embodiments will emit visible light energy at wavelengths ranging between 400-495 nm (the blue color range) at a radiant power between 1-20 mW.

### A Source Of Electric Current:

It is required that a portable and replenishable source of on-demand direct electrical current exist as a component part of the apparatus and system of the present invention. The therapeutic treatment method and system provided by the instant invention is intended to deliver a specific energy dosage (measured in Joules), which is a function of power (in wattage) and time (in seconds), and which is deemed to be efficacious for each therapeutic treatment.

In the preferred embodiments shown by Figs. 5 and 6 respectively, a 1.5 volt dry cell battery is employed to power the diodes. The respective embedded programs will enable components to deliver therapeutic energy as follows:
◊ 11-12 Joules/cm² for the 633 nm LED embodiment;
◊ 7-8 Joules/cm² for the 655 nm low level laser embodiment; and
◊ 11-13 Joules/cm² for the 810 nm pulsed LED (before pulsed mode duty-cycle).

It is also expected and intended that there will be other alternative embodiments with different combinations of these components and which would require different configurations of power, energy dosage and treatment time.

The power supply typically will convey energy in the form of direct electric current; and adequate quantities of electric current can be repeatedly conveyed from either from a single battery source or from a combination of several dry cells joined together in series or parallel. In some other desirable embodiments, the source of electric power will be in the form of a rechargeable direct current battery unit (rechargeable from ordinary household alternating current receptacles) or as alternating current (AC) via a power adaptor.

As to positioning, in all preferred embodiments, the power source is a discrete entity which is held and contained entirely within the internal confines of the controller assembly 28. In less preferred embodiments, however, the source of electric current can be a self-contained, separate and free standing unit which is in electric communication with the controller assembly via an electrical cable and connector module linkage.

### A Process Controller Assembly:

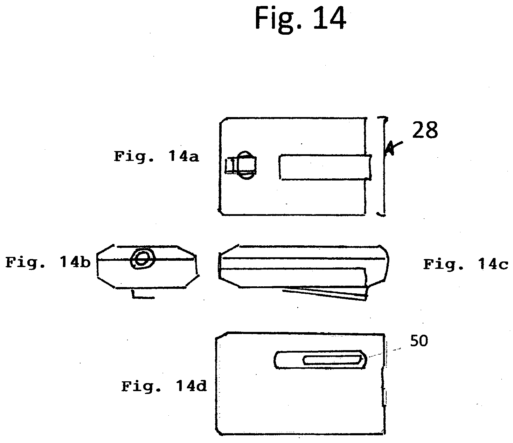

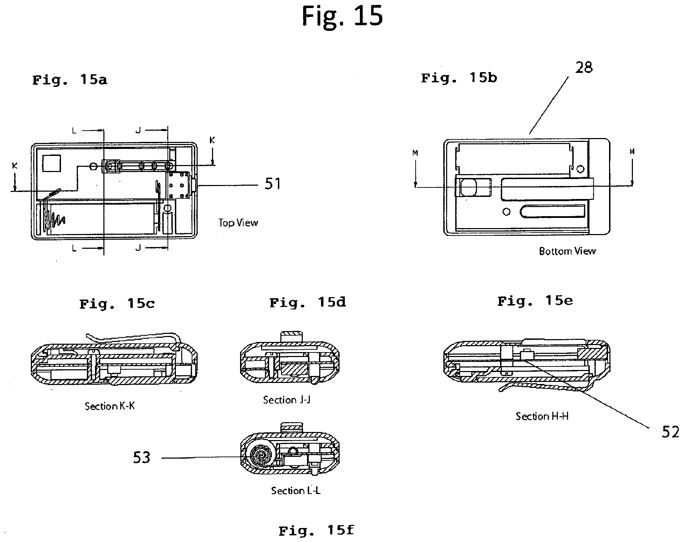

The process controller assembly is a portable unit component having three structural features. Thus, as illustrated by Figs. 14 and 15 respectively, each process controller assembly will
include:
(i) A receiving circuit for receipt of such direct electrical current as is transferred to the controller assembly from the electric source;
(ii) A central processing unit (CPU) for controlling and directing the flow of such electrical current as is received by the controller assembly over time; and
(iii) A delivery circuit for delivering direct electrical current from the controller assembly to another component.

Equally important, it is intended and expected that the process controller assembly will be electrically linked to the other essential components of the apparatus and thus typically will
also have:
(a) At least one connector in electrical communication with the source of electrical current for on-demand transfer of electric current to the controller assembly; and
(b) At least one connector in electrical communication with the light generating unit for on-demand conveyance of electric current from the controller assembly to the light generating unit(s).

These connectors typically are formed as insulated copper wire cables and jack modules that allow for quick and easy linkage and electrical communication with both the power source and the light generating unit(s).

In all embodiments of the apparatus, the process controller assembly will not operate in the absence of a source of electric current. In addition, the controller assembly, besides switching off the unit after a predetermined time, is mainly a circuitry which provides power to drive the light generating unit properly and efficiently. The controller also ensures that the power delivered to the light generating unit is consistent. It therefore desirably monitors the battery strength, and switches off the unit if the battery if it is unable to supply sufficient power to drive the circuitry properly.

Accordingly, as shown by Figs. 5-6, 14a-14d, and 15a-15f respectively, the preferred process controller assembly 28 is dimensionally small in size, light in weight, and portable. It preferably has fixed dimensions which are no larger than an average shirt pocket - i.e., approximately 4.5 inches in length, by 4.5 inches in width, by 1 inch in depth; and is formed of a resilient material such as a moldable thermoplastic. Preferred embodiments of the controller assembly typically include a central processing unit (CPU) in a circuit board 52 which is able to control and direct the flow of electric current in dosage, power, and time from the power source, which is a single AA dry cell battery in the preferred embodiment 53, to the configured irradiation lens 39.

Note also that in the preferred embodiment shown by Figs. 5 and 6, the source of direct electric current lies internally and is contained within the interior spatial volume of the controller assembly; and appears as the electric battery 29 (dry cell or rechargeable unit). In this instance, the controller assembly 28 also has a socket adapted for the attachment of an insulated copper wire cable and modular jack connector 51, whose other end is joined to the light generating unit 38 disposed within the hollow casing 39 illustrated by Fig. 8.

The controller assembly 28 illustrated by Figs. 14a-14d and 15a-15f as a whole is able to deliver the required dosage from the light diode repeatedly over time, which is sufficient to achieve consistent neurostimulation of the brain. Also for portability, a typical battery source of electric energy provides
direct current at 1.5 volts. However, subject to the circuit to the type of light source being used, a broader range of direct current voltages is acceptable.

The central processing unit ("CPU") of the controller assembly is able to regulate light energy power output at 1-20 mW or more. When it is regulated, the power is typically fixed. Thus, in the low level laser embodiment, it is typically about 5mW; whereas in the LED embodiments of continuous wave, it is typically about 8mW. These light energy power outputs result in the emitted light of the apparatus being therapeutically effective after a treatment time of only 25 or 30 minutes per session duration for the preferred embodiments.

It is intended and expected that any conventionally known and interchangeable electric cables and connectors will be used to link the controller assembly to the irradiation lens. This also provides a distinct advantage and benefit to the user - i.e., the option to exchange one configured irradiation lens (able to transmit light at a first wavelength) for another irradiation lens (able to transmit light at a second and different wavelength); and thereby permits the use of different lasers and alternative light emitting diodes able to deliver different wavelengths of visible and invisible light energy with one single controller assembly.

In another embodiment, the controller assembly may have controls to provide more delivery and operating options to offer more versatility. This particular advantage and benefit is provided through the selection of various preconfigured settings on the controller to match the type of light and its wavelength, improve user-interface and reduce errors. This mode and manner of exchanging the light generating units at will or as needed allows the therapeutic use of different light ranges and alternative
light wavelengths by a single patient without any need for purchasing multiple treatment systems or more than one apparatus.

### The Mobile Smart Phone Option:

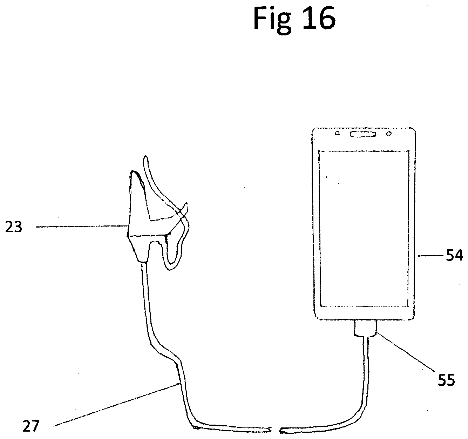

In another embodiment, the function of the controller assembly 28 may be replaced by a smart phone operating on one of the popular mobile platforms, - and which may include those from Apple, Android, Blackberry and Windows as illustrated by Fig.16. The applicator assembly 23 will now be connected via a cable (formed of similar materials as for the other embodiments 27) to the smart phone 54 instead with a discrete controller unit. The smart phone 54 carries an downloadable software application ("App") that would largely duplicate the software functions in the controller assembly 28. A modified attachment containing interface processing software in a computer chip 55 will provide an interface between the existing applicator and the proprietary smart phone platform.

With this embodiment, the user need not carry an additional or separate controller unit, and yet the "App" will also contain more software controls and graphic interfaces.

### VII. Notable Features And Capabilities Of The Apparatus

The apparatus provides a number of unique attributes, properties, and capabilities. Among them are the following:
1. The apparatus and system can deliver light energy over a variety of selected wavelengths to achieve therapeutic outcomes of the brain and the neural system.
2. The apparatus and system have very low electric power requirements.
3. The apparatus and system provide an easy to use applicator which can be clipped to the external wall of a nostril while concomitantly inserting an encased solid state electronic light source (such as the light emitting diode or a low level laser diode) within the nasal cavity to
   deliver the light therapy.
4. The apparatus and system overcome the disadvantages of the prior art and the limitations of conventional technologies, particularly with regards to portability and self-administration. It also markedly differs from known system as to the method by which light therapy is delivered to the brain.
5. The apparatus and system is able to illuminate the various targeted areas of the brain by way of directing the light rays and light wavelengths. In this respect, the wavelengths of light are pre-selected so as to the desired penetration into the brain materials. This results in improved outcomes for respective neural diseases and disorders.
6. The apparatus and system will direct dispersed light waves and particles and achieve wide or focused light coverage of the targeted areas of the brain through the interior nasal cavity , with minimum leakage through the anterior nasal region.
7. The apparatus and system separate the delivery of light energy from the processing and power controller assembly for both hygiene and cost saving purposes; and also potentially provides for the interchange and substitution of different light generating units with a single processing controller assembly that will convey the appropriate power dosage for this purpose, as well as to interface with a mobile smart phone.

### VIII. The Method Of Therapeutic Treatment

The method for performing non-invasive irradiation light therapy within a nasal cavity in order to achieve brain neurostimulation in a living mammalian subject typically comprises the following steps and actions:
Step 1: obtaining a light energy-emitting apparatus comprised of a configured irradiation lens including
a portable hollow casing having fixed dimensions, a sized internal spatial volume, and an external surface configuration suitable for in-vivo insertion into the nasal cavity space of a nostril without causing substantial impairment to the subject's ability to breathe and without invading the nasal tissues of the living subject, said portable casing being comprised of
   (i) a light energy transmitting material which forms at least a portion of the configured external surface for said hollow casing,
   (ii) at least one light generating unit entirely housed and contained within said internal spatial volume of said hollow casing and which is capable of generating light energy of at least one preselected wavelength selected from the group consisting of near infrared red light wavelengths and visible red light wavelengths, at a predetermined energy intensity and for a preset time duration on-demand sufficient to penetrate through the nasal tissues and to pass into the brain,
   whereby said configured irradiation lens can emit light energy in any desired direction within the nasal cavity after in-vivo insertion and achieve passage of said emitted light energy from the nasal cavity into at least one portion of the brain in-vivo;
self-administrable applicator means adapted for support of said configured irradiation lens and for at will placement of said light transmitting external surface of said configured irradiation lens at a fixed position and desired irradiation direction within a nostril adjacent to the internal lining of a subject's nasal cavity;
a portable controller assembly able to control on-demand delivery of light energy from said configured irradiation lens through the subject's nasal tissues into at least one portion of the brain in-vivo, said controller assembly including
   (α) a portable and replenishable power source of on-demand direct electrical current,
   (β) a central processing unit for controlling and directing the flow of such direct electrical current, and
   (γ) at least one connector in electrical communication with said configured irradiation lens for on-demand conveyance of direct electrical current from said controller assembly to said light generating unit.
Step 2: Placing a transparent external surface of said configured irradiation lens within a nostril at a desired fixed position adjacent to the internal lining of a subject's nasal cavity such that light energy emitted by said configured irradiation lens will penetrate through the subject's nasal tissues and pass into at least one portion of the brain in-vivo. And
Step 3: Causing said light generating unit of said positioned configured irradiation lens to generate light energy of at least one preselected wavelength selected from the group consisting of near infrared red light wavelengths and visible red light wavelengths, at a predetermined energy intensity and for a preset time duration on-demand sufficient to penetrate through the subject's nasal tissues and to pass into the brain such that neurostimulation of at least one portion of the brain is achieved.

The treatment methodology as recited above is illustrated by Figs. 10-16 respectively. As seen in Fig. 10, one properly inserts the configured irradiation lens 35 using the self-administrable applicator means 23 into the nasal cavity space 2 of the human nose; and then causes direct electric current to be conveyed from its source to the process controller assembly (not shown) and then to the light unit(s) 35 disposed within the hollow casing 39. This will cause the light unit 45 to generate and transmit light waves and particles 46 of a pre-chosen wavelength.

In the preferred laser light embodiments, the transmitted light waves and particles 46 are directed and deflected by the casing wall 40 and the micro-lens 44 that is capped over the light generating unit 45 within the hollow casing 35. This is illustrated best by Figs. 11 and 12 respectively.

By this process, the light waves and particles 46 emanating from the configured irradiating lens 35 become focused, aimed, and directed towards entering the various regions of the brain, as illustrated in Fig. 1. The aimed light energy from the light generating unit 45 is largely deflected towards the internal section of the nasal cavity; and such light as might pass through the entire nasal cavity 2 and leaked light is then reflected and redirected back into the nasal cavity space by the reflective white opaque material of the nose clip 40. Thus, as shown by Fig. 12, most of the initially generated light is captured and aimed (by deflection and reflection) into the nasal cavity 2.

In these embodiments, the process and power controller assembly as a whole is able to deliver a dosage of the desired energy measured in Joules/cm², which is sufficient to achieve consistent therapeutic stimulation of the brain. Also, the battery source of electric energy within the controller assembly provides direct current at a constant 1.5 volts and the CPU of the controller assembly is able to regulate light energy power output consistently at 1-20 mW. This results in the emitted light of the apparatus being therapeutically effective after a treatment time of only 25 or 30 minutes duration.

In the alternative, the functions and work of the power controller may alternatively be performed by a smart phone with the appropriate downloaded "App".

The present invention is not limited in form nor restricted in scope except by the claims appended hereto.

## Claims

1. A self-administrable system for performing non-invasive neurostimulation therapy of the brain via a nasal cavity of a living mammalian on-demand, said self-administrable non-invasive neurostimulation system comprising:
a configured irradiation lens (24, 35, 39) including
a portable hollow casing (35) having fixed dimensions, a sized internal spatial volume, and an external surface configuration suitable for in-vivo insertion into the nasal cavity space of a nostril without causing substantial impairment to the subject's ability to breathe and without invading the nasal tissues of the living subject, said portable casing (28) being comprised of
(i) a light energy transmitting material which forms at least a portion of the configured external surface for said hollow casing (35),
(ii) at least one light generating unit (29, 38, 45) entirely housed and contained within said internal spatial volume of said hollow casing (35) and which is capable of generating light energy of at least one preselected wavelength selected from the group consisting of near infrared red light wavelengths and visible red light wavelengths, at a predetermined energy intensity and for a preset time duration on-demand sufficient to penetrate through the nasal tissues and to pass into the brain,
whereby said configured irradiation lens (24, 35, 39) can emit light energy in any desired direction within the nasal cavity after in-vivo insertion and achieve passage of said emitted light energy from the nasal cavity into at least one portion of the brain in-vivo;
self-administrable applicator means adapted for support of said configured irradiation lens (24, 35, 39) and for at will placement of said light transmitting external surface of said configured irradiation lens (24, 35, 39) at a fixed position and desired irradiation direction within a nostril adjacent to the internal lining of a subject's nasal cavity;
a portable controller assembly (28) able to control on-demand delivery of light energy from said configured irradiation lens (24, 35, 39) through the subject's nasal tissues into at least one portion of the brain in-vivo, said controller assembly (28) including
(α) a portable and replenishable power source of on-demand direct electrical current,
(β) a central processing unit for controlling and directing the flow of such direct electrical current, and
(γ) at least one connector in electrical communication with said configured irradiation lens (24, 35, 39) for on-demand conveyance of direct electrical current from said controller assembly (28) to said light generating unit (29, 38, 45);
wherein the system is configured to operate at the following parameters:
(a) the light energy has a wavelength of about 810 nm;
(b) the light energy is emitted for a duration of about 10 to 30 minutes;
(c) the controller assembly (28) regulates the power to about 1 to 20 mW;
(d) the light energy is about 5 to 20 Joules; and
(e) the light energy is pulsed at a frequency of 4 to 10 Hz.

2. The system for performing non-invasive neurostimulation therapy of the brain as recited in claim 1, wherein the light energy is emitted for a duration of about 25 to 30 minutes.

3. The system for performing non-invasive neurostimulation therapy of the brain as recited in claim 1 further comprising at least one power switch which engages and disengages the transfer of direct electrical current from said controller assembly (28) to said configured irradiation lens(24, 35, 39).

4. The system for performing non-invasive neurostimulation therapy of the brain as recited in claim 1 further comprising at least one timer which limits the transfer of direct electrical current from said controller assembly (28) to said configured irradiation lens (24, 35, 39) to a pre-chosen time period.

5. The system for performing non-invasive neurostimulation therapy of the brain as recited in claim 1 further comprising a micro-lens (44) housed within said hollow casing (35).

6. The system for performing non-invasive neurostimulation therapy of the brain as recited in claim 1 wherein said light generating unit (29, 38, 45) disposed within said internal spatial volume of said hollowing casing is one selected from the group consisting of a laser unit and an a light emitting diode unit.

7. The system for performing non-invasive neurostimulation therapy of the brain as recited in claim 1 wherein said power source of on-demand direct electrical current is 1.5 volt battery.

8. The system for performing non-invasive neurostimulation therapy of the brain as recited in claim 1 further comprising applicator means which can be clipped to the external wall of a subject's nostril while concomitantly inserting said configured irradiation lens (24, 35, 39) within the nasal cavity to deliver the light energy.

## Patentansprüche

1. Selbstverwaltendes System zur Durchführung einer nichtinvasiven Neurostimulationstherapie des Gehirns über eine Nasenhöhle eines lebenden Säugetiers bei Bedarf, wobei das selbstverwaltende nichtinvasive Neurostimulationssystem umfasst:
eine ausgelegte Bestrahlungslinse (24, 35, 39), umfassend
ein tragbares hohles Gehäuse (35) mit festen Abmessungen, einem großen Innenraumvolumen und einer Außenseitenauslegung, die zum In-vivo-Einsatz in den Nasenhöhlenraum eines Nasenlochs geeignet ist, ohne dabei die Fähigkeit des Subjekts zum Atmen wesentlich zu beeinträchtigen und ohne dabei in die Nasengewebe des lebenden Subjekts einzudringen, wobei das tragbare Gehäuse (28) aus
(i) einem lichtenergieübertragenden Material, das mindestens einen Abschnitt der ausgelegten Außenfläche für das hohle Gehäuse (35) bildet,
(ii) mindestens einer lichterzeugenden Einheit (29, 38, 45), die vollständig in dem Innenraumvolumen des hohlen Gehäuses (35) untergebracht und enthalten ist, und die Lichtenergie mit mindestens einer vorausgewählten Wellenlänge, die aus der Gruppe bestehend aus Nahinfrarotlichtwellenlängen und Wellenlängen sichtbaren Rotlichts ausgewählt ist, mit einer vorbestimmten Energieintensität und für eine voreingestellte Zeitdauer bei Bedarf erzeugen kann, die ausreicht, um die Nasalgewebe zu durchdringen und ins Gehirn überzugehen, besteht,
wodurch die ausgelegte Bestrahlungslinse (24, 35, 39) nach dem In-vivo-Einsatz Lichtenergie in jede gewünschte Richtung innerhalb der Nasenhöhle emittieren kann und den Übergang der emittierten Lichtenergie aus der Nasenhöhle in mindestens einen Abschnitt des Gehirns in-vivo erreicht;
ein selbstverwaltendes Applikatormittel, das zum Tragen der ausgelegten Bestrahlungslinse (24, 35, 39) und zur beliebigen Platzierung der lichtübertragenden Außenfläche der ausgelegten Bestrahlungslinse (24, 35, 39) in einer festen Position und zur gewünschten Bestrahlungsrichtung in einem Nasenloch neben der Innenauskleidung der Nasenhöhle eines Subjekts angepasst ist;
eine tragbare Steuerungsanordnung (28), die die Bedarfsabgabe von Lichtenergie aus der ausgelegten Bestrahlungslinse (24, 35, 39) durch die Nasalgewebe des Subjekts in mindestens einen Abschnitt des Gehirns in-vivo steuert, wobei die Steuerungsanordnung (28) umfasst
(α) eine tragbare und erneuerbare Stromquelle für Bedarfsgleichstrom,
(β) eine Zentraleinheit zum Steuern und Leiten des Flusses aus einem derartigen Gleichstrom, und
(γ) mindestens einen Anschluss in elektrischer Kommunikation mit der ausgelegten Bestrahlungslinse (24, 35, 39) für den Bedarfstransport des Gleichstroms von der Steuerungsanordnung (28) an die lichterzeugende Einheit (29, 38, 45);
wobei das System dazu ausgelegt ist, bei den folgenden Parametern zu arbeiten:
(a) die Lichtenergie weist eine Wellenlänge von etwa 810 nm auf;
(b) die Lichtenergie wird für eine Dauer von etwa 10 bis 30 Minuten emittiert;
(c) die Steuerungsanordnung (28) steuert den Strom auf ungefähr 1 bis 20 mW;
(d) die Lichtenergie beträgt ungefähr 5 bis 20 Joule; und
(e) die Lichtenergie ist auf eine Frequenz von 4 bis 10 Hz gepulst.

2. System zur Durchführung einer nichtinvasiven Neurostimulationstherapie des Gehirns nach Anspruch 1, wobei die Lichtenergie für eine Dauer von ungefähr 25 bis 30 Minuten emittiert wird.

3. System zur Durchführung einer nichtinvasiven Neurostimulationstherapie des Gehirns nach Anspruch 1, das ferner mindestens einen Stromschalter umfasst, der den Transfer von Gleichstrom von der Steuerungsanordnung (28) an die ausgelegte Bestrahlungslinse (24, 35, 39) einschaltet und ausschaltet.

4. System zur Durchführung einer nichtinvasiven Neurostimulationstherapie des Gehirns nach Anspruch 1, das ferner mindestens einen Zeitgeber umfasst, der den Transfer von Gleichstrom von der Steuerungsanordnung (28) an die ausgelegte Bestrahlungslinse (24, 35, 39) auf einen vorausgewählten Zeitraum begrenzt.

5. System zur Durchführung einer nichtinvasiven Neurostimulationstherapie des Gehirns nach Anspruch 1, das ferner eine Mikrolinse (44) umfasst, die in dem hohlen Gehäuse (35) untergebracht ist.

6. System zur Durchführung einer nichtinvasiven Neurostimulationstherapie des Gehirns nach Anspruch 1, wobei die lichterzeugende Einheit (29, 38, 45), die in dem Innenraumvolumen des hohlen Gehäuses angeordnet ist, eine aus der Gruppe bestehend aus einer Lasereinheit und einer Leuchtdiodeneinheit ausgewählte Einheit ist.

7. System zur Durchführung einer nichtinvasiven Neurostimulationstherapie des Gehirns nach Anspruch 1, wobei die Stromquelle für den Bedarfsgleichstrom eine 1,5 Volt Batterie ist.

8. System zur Durchführung einer nichtinvasiven Neurostimulationstherapie des Gehirns nach Anspruch 1, das ferner ein Applikatormittel umfasst, das an die Außenwand eines Nasenlochs eines Subjekts geklemmt werden kann, während gleichzeitig die ausgelegte Bestrahlungslinse (24, 35, 39) in die Nasenhöhle eingesetzt wird, um die Lichtenergie abzugeben.

## Revendications

1. Système à administration automatique permettant de réaliser à la demande une thérapie de neurostimulation non invasive du cerveau par l'intermédiaire de la cavité nasale d'un mammifère vivant, ledit système de neurostimulation non invasive à administration automatique comprenant :
une lentille de rayonnement (24, 35, 39) configurée comprenant
un boîtier creux portatif (35) ayant des dimensions fixes, un volume spatial interne dimensionné, et une configuration de surface externe adaptée à une introduction in vivo dans l'espace de cavité nasale d'une narine sans trop gêner la capacité du sujet à respirer et sans envahir les tissus nasaux du sujet vivant, ledit boîtier portatif (28) étant composé de
(i) un matériau de transmission d'énergie lumineuse qui forme au moins une partie de la surface externe configurée pour ledit boîtier creux (35),
(ii) au moins une unité de génération de lumière (29, 38, 45) entièrement logée et contenue dans ledit volume spatial interne dudit boîtier creux (35) et qui est susceptible de générer une énergie lumineuse d'au moins une longueur d'onde présélectionnée choisie dans le groupe constitué par les longueurs d'onde de lumière rouge dans le proche infrarouge et des longueurs d'onde de lumière rouge visible, à une intensité d'énergie prédéfinie et pendant une durée prédéfinie à la demande suffisantes pour pénétrer par les tissus nasaux et pour passer dans le cerveau,
moyennant quoi ladite lentille de rayonnement configurée (24, 35, 39) peut émettre une énergie lumineuse dans n'importe quelle direction souhaitée dans la cavité nasale après une introduction in vivo et obtenir un passage de ladite énergie lumineuse depuis la cavité nasale vers au moins une partie du cerveau in vivo ;
des moyens d'application à administration automatique conçus pour supporter ladite lentille de rayonnement configurée (24, 35, 39) et pour positionner au besoin ladite surface externe de transmission de lumière de ladite lentille de rayonnement configurée (24, 35, 39) en une position fixe et à une direction de rayonnement souhaitée dans une narine adjacente à la muqueuse interne de la cavité nasale d'un sujet ;
un ensemble organe de commande portatif (28) pouvant commander une distribution à la demande d'énergie lumineuse à partir de ladite lentille de rayonnement configurée (24, 35, 39) à travers les tissus nasaux du sujet dans au moins une partie du cerveau in vivo, ledit ensemble organe de commande (28) comprenant
(α) une source portative et renouvelable de courant électrique continu à la demande,
(β) une unité centrale de traitement permettant de commander et de diriger le flux d'un tel courant électrique continu, et
(γ) au moins un connecteur en communication électrique avec ladite lentille de rayonnement configurée (24, 35, 39) pour un transport à la demande du courant électrique continu depuis ledit ensemble organe de commande (28) vers ladite unité de génération de lumière (29, 38, 45) ;
dans lequel le système est conçu pour fonctionner aux paramètres suivants :
(a) l'énergie lumineuse a une longueur d'onde d'environ 810 nm ;
(b) l'énergie lumineuse est émise pendant une durée d'environ 10 à 30 minutes ;
(c) l'ensemble organe de commande (28) régule la puissance d'environ 1 à 20 mW ;
(d) l'énergie lumineuse est d'environ 5 à 20 joules ; et
(e) l'énergie lumineuse est pulsée à une fréquence de 4 à 10 Hz.

2. Système permettant de réaliser une thérapie de neurostimulation non invasive du cerveau selon la revendication 1, dans lequel l'énergie lumineuse est émise pendant une durée d'environ 25 à 30 minutes.

3. Système permettant de réaliser une thérapie de neurostimulation non invasive du cerveau selon la revendication 1 comprenant en outre au moins un commutateur d'alimentation qui active et désactive le transfert du courant électrique continu depuis ledit ensemble organe de commande (28) vers ladite lentille de rayonnement configurée (24, 35, 39).

4. Système permettant de réaliser une thérapie de neurostimulation non invasive du cerveau selon la revendication 1 comprenant en outre au moins un chronomètre qui limite le transfert du courant électrique continu depuis ledit ensemble organe de commande (28) vers ladite lentille de rayonnement configurée (24, 35, 39) à une période temporelle préalablement choisie.

5. Système permettant de réaliser une thérapie de neurostimulation non invasive du cerveau selon la revendication 1 comprenant en outre une microlentille (44) logée dans ledit boîtier creux (35).

6. Système permettant de réaliser une thérapie de neurostimulation non invasive du cerveau selon la revendication 1 dans lequel ladite unité de génération de lumière (29, 38, 45) disposée dans ledit volume spatial interne dudit boîtier creux est sélectionnée dans le groupe constitué par une unité laser et une unité à diode électroluminescente.

7. Système permettant de réaliser une thérapie de neurostimulation non invasive du cerveau selon la revendication 1 dans lequel ladite source d'alimentation en courant électrique continu à la demande est une batterie de 1,5 volt.

8. Système permettant de réaliser une thérapie de neurostimulation non invasive du cerveau selon la revendication 1 comprenant en outre des moyens d'application qui peuvent être fixés à la paroi externe de la narine d'un sujet tout en introduisant simultanément ladite lentille de rayonnement configurée (24, 35, 39) dans la cavité nasale pour distribuer l'énergie lumineuse.
